# EUROPEAN PATENT SPECIFICATION

(11) **EP 2 240 125 B1**
(45) Date of publication and mention of the grant of the patent: **27.06.2012**
(21) Application number: 09704862.3
(22) Date of filing: 23.01.2009
(51) Int. Cl.: A61F 2/84

(54) **APPARATUS AND METHOD FOR LOADING AND DELIVERING A STENT HAVING IMPROVED HANDLES TO CONTROL RELATIVE CATHETER COMPONENT MOVEMENT**
GERÄT UND VERFAHREN ZUM LADEN UND ABGEBEN EINES STENTS MIT VERBESSERTEN HANDGRIFFEN ZUR KONTROLLE DER RELATIVEN BEWEGUNG VON KATHETERKOMPONENTEN
APPAREIL ET PROCÉDÉ DE CHARGEMENT ET DE LARGAGE D'UN STENT, AYANT DES POIGNÉES AMÉLIORÉES POUR COMMANDER UN MOUVEMENT RELATIF DES COMPOSANTS DE CATHÉTER

(30) Priority: 24.01.2008 US 23233 P
(43) Date of publication of application: 20.10.2010
(73) Proprietor: Boston Scientific Scimed, Inc., Maple Grove, MN 55311 (US)
(72) Inventor: GOLDEN, John, Norton MA 02766 (US); WOOD, Mark, Shrewsbury MA 01545 (US); MCWEENEY, John, Brighton MA 02135 (US); LEANNA, Gary, Holden MA 01520 (US)
(74) Representative: Vossius & Partner
(86) International application number: PCT/US2009/031746
(87) International publication number: WO 2009/094490

(56) References cited:
- WO-A-99/39661
- US-A- 5 954 729
- US-A1- 2007 118 207
- US-A1- 2007 270 932

## Description

### CROSS-REFERENCE TO REFLATED APPLICATIONS

This Application claims the benefit of U.S. Provisional Application No. 61/023,233, filed January 24, 2008.

### FIELD OF THE INVENTION

This invention relates to a delivery assembly and method for transporting, loading and delivering a stent in a bodily passageway (this last method is not part of the invention). More particularly, this invention relates to systems and methods for loading and delivering (this last method is not part of the invention) radially distensible stents, including polymeric and non-polymeric stents.

### BACKGROUND OF THE INVENTION

An intraluminary prosthesis is a medical device used in the repair and/or treatment of diseases in various body vessels is a stent. A stent is generally a longitudinal tubular device formed of biocompatible material useful to open and support various lumens in the body. For example, stents may be used in the bodily vessel, such as in the coronary or peripheral vasculature, esophagus, trachea, bronchi colon, biliary tract, urinary tract, prostate, brain, as well as in a variety of other applications in the body. These devices are implanted within the vessel to open and/or reinforce collapsing or partially occluded sections of the lumen.

Stents generally include an open flexible configuration such as helically wound coils with undulations or zig-zags therein, slotted stents, ring stents, braided stents and open mesh wire stents, to name a few. Super-elastic materials and metallic shape memory materials have also been used to form stents. This flexible configuration allows the stent to be inserted in a radially compressed state through curved vessels. Once properly positioned adjacent to the damaged vessel, the stent is radially expanded so as to support and reinforce the vessel. Radial expansion of the stent may be accomplished by inflation of a balloon attached to the catheter or the stent may be of the self-expanding variety which will radially expand once deployed. Once the stent is implanted, the delivery catheter is withdrawn from the patient.

With any of these systems, a sheath may be provided over the distal end of the catheter to protect the components on the distal end, such as a balloon, a stent, an array of electrodes, and the like. In some embodiments, the sheath may be advanced distally over the proximal end of the catheter until it covers the distal end and its components, or, alternatively, the distal end of the catheter may be introduced into the sheath, and advanced until it is proximate the distal end of the sheath. Once the distal end of the catheter is properly positioned at a desired location within a body lumen, the sheath may be retracted to expose the distal end of the catheter. After treatment, the sheath may be advanced back over the distal end of the catheter or the catheter may be withdrawn back into the sheath, and the entire device withdrawn from the patient.

To cause the sheath to retract, the proximal end of the sheath outside the patient may simply be pulled while holding the catheter in a fixed position. This, however, may not provide very precise control of the retraction of the sheath. Moreover, in such devices, it is possible to advance the sheath in the distal direction during and after deployment of the device, such as a stent, on the distal end of the catheter. This distal movement may result in the improper placement and unwanted movement of the deployed device. This distal movement of the sheath is particularly problematic in the deployment of stents or other tubular prostheses. Accordingly, there is a need for more intuitive, simpler, and/or less expensive devices for controlling catheter-sheath systems.

Although stent delivery systems are well-known in the art, the assembly of such delivery systems is often complicated. Unlike most metallic self-expanding stents, the plastic stents have a tendency to permanently deform or lose some of their ability to self-expand when stored in a compressed state for a prolonged period of time. These stents are therefore preferably loaded into the stent delivery system shortly before being implanted in a patient.

However, such loading step just prior to the actual surgery often involves numerous steps and requires the use of multiple components (e.g., tools and fixtures) that are not part of the stent delivery system. Also, even with these added devices, the practitioner is often required to finish the loading process by pushing the stent into the delivery system by hand. Loading a stent in this way is therefore often difficult, time-consuming and has the potential to damage the stent. Accordingly, there is a need for simplified methods of on-site loading of a stent into stent delivery systems, while minimizing the risk of damaging the stent in the process.

### SUMMARY OF THE INVENTION

The present invention is directed to a system for delivering a self-expanding stent into a body lumen as disclosed in the appended claims. In particular, the present invention relates to an assembly as disclosed in claims 1-14 and a method for loading a stent in combination with a stent delivery catheter device as disclosed in claims 15, as well as provides examples of overall stent delivery systems.

In one aspect of the present invention, a stent loading and deployment device may be provided. The device may include an axially elongated external member having opposed proximal and distal ends; and an axially elongated inner member having opposed proximal and distal ends and slidably disposed within the axially elongated external member. When the distal ends of the axially elongated external member and the axially elongated inner member are axially aligned, a stent deployment region can be defined there in between. An intermediate member having opposed proximal and distal ends can be slidably disposed between the external member and the inner member. In some embodiments, the distal end of the intermediate member may be slidable to a distal position past the distal end of the external member for receiving a stent and may be further slidable toward the proximal end of the external member to a location past the stent deployment region for disengagement of the stent from the intermediate member. The external member, the inner member and/or the intermediate member may be axially movable or slidable independently of each other. They may also be axially movable or slidable in concert in either total or in different combinations of pairs. For example, the distal end of the intermediate member may be slidable to a distal position past the distal end of the external member while the positions of the inner member and the external member arc kept constant or relatively constant. The intermediate member may be further slidable toward the proximal end of the external member to a location past the stent deployment region while the positions of the inner member and the external member are kept constant or relatively constant.

The device includes a stent basket having opposed proximal and distal ends. The proximal end may be securely disposed to the distal end of the intermediate member. The stent basket may have a truncated-conical shape, outwardly diverging in a distal direction from its proximal end. The stent basket may be a thin film which can collapse such that the stent basket may be slidably contained within the external member, or may be a radially distensible member which can collapse such that the stent basket may be slidably contained within the external member. In some embodiments, the stent basket may be composed of a polymeric material. The stent basket may include, in part or substantially, braided polymeric filaments. The braided filaments may be contained within a thin polymeric film. The intermediate member may be an elongate tubular device. The stent basket may comprise metals, polymers, or combinations of both.

The device may further include a tubular band disposed toward the distal end of the inner member for releasably securing a stent in the stent deployment region between the inner member and the external member. In some embodiments, the external member can be slidable toward a proximal position for releasing the stent from the stent deployment region. Typically, the external member may slide while the inner member and the stent basket may be fixed or not in substantial movement.

The device may further include a distal handle disposed at the proximal end of the external member; a proximal handle may be disposed at the proximal end of the inner member; and an intermediate handle may be disposed at the distal end of the intermediate member. The intermediate handle may be axially disposed between the distal handle and the proximal handle. The distal handle may be axially disposed distal to the proximal end of the inner member. The handles may separated, mechanically mated, including temporarily mated or locked, and/or integrated to allow independent or non-independent axial movement or sliding of the external member, the inner member and the intermediate member.

The device of this aspect can be useful containing and releasing a radially distensible stent. The radially distensible stent may be a polymeric stent, including a braided stent. A graft, such as a covering, a liner, a film, a coating and combinations thereof, may be disposed over at least a portion of the stent. In some embodiments, the stent can be a braided polymeric stent and the graft may be a silicone coating or film. Further, the stent may be a multi-component stent (graft separate from stent) and may comprise metal.

In another aspect of the present invention, a stent loading and deployment system can be provided. The system includes a radially distensible stent; an external member having opposed proximal and distal ends; an inner member having opposed proximal and distal ends and slidably disposed within the external member, wherein, when the distal ends of the external member and the inner member can be axially aligned, a stent deployment region being defined there in between; and an intermediate member having opposed proximal and distal ends and slidably disposed between the external member and the inner member; wherein the distal end of the intermediate member may be slidable to a distal position past the distal end of the external member for receiving the stent and can be further slidable toward the proximal end of the external member to a location past the stent deployment region for disengagement of the stent from the intermediate member.

A method for loading a stent into a delivery and deployment may also be provided with under the present invention, such method includes (i) providing a radially distensible stent having opposed proximal and distal ends; providing a delivery deployment device, the device including an external member having opposed proximal and distal ends; an inner member having opposed proximal and distal ends and slidably disposed within the external member, wherein, when the distal ends of the external member and the inner member may be axially aligned, a stent deployment region may be defined there in between; a stent basket having opposed proximal and distal ends, wherein the proximal end of the stent basket can be securely disposed to the distal end of the intermediate member; (ii) axially moving or sliding the distal end of the intermediate member to a distal position past the distal end of the external member; (iii) engaging the proximal end of the stent with the stent basket; (iv) axially moving or sliding the stent and the intermediate member toward the proximal end of the external member to radially compress the stent within the stent deployment region; and (v) axially moving or sliding the stent basket to a location past the stent deployment region for disengagement of the stent from the intermediate member.

The method may further include providing a tubular band disposed toward the distal end of the inner member for releasably securing the stent in the stent deployment region between the inner and external members. Moreover, the method, in an example not part of the invention, may further include axially moving or sliding the external member toward a proximal position for releasing the stent from the stent deployment region. The method may yet further include providing a distal handle disposed at the proximal end of the external member; providing a proximal handle disposed at the proximal end of the inner member; and providing an intermediate handle disposed at the proximal end of the intermediate member, wherein independent axial movement of the external member, the inner member or the intermediate member can be achieved by manual manipulation of the handles.

Further aspects are described as follows :

These and other objectives, features, and advantages of this invention will become apparent from the following detailed description of illustrative embodiments thereof, which is to be read in connection with the accompanying drawings.

Document US 2007/0270 932 discloses the preamble of claim 1.

### BRIEF DESCRIPTION OF THE DRAWINGS

Fig. 1 is a cross sectional view of an embodiment of the present invention.
Fig. 2 is a cross-sectional view of distal part of the embodiment as shown in FIG. 1.
Fig. 3 is a top plan view of a stent of the present invention.
Fig. 4 is a cross sectional view of another embodiment of the stent of the present invention.
Fig. 5 is a cross sectional view of yet another embodiment of the stent of the present invention.
Fig. 6 is a cross sectional view of further yet another embodiment of the stent of the present invention.
Fig. 7 is a side plan view of further yet another embodiment of the stent of the present invention.
Fig. 8 is a side plan view of further yet another embodiment of the stent of the present invention.
Fig. 9 is a cross-sectional view of the embodiment shown in FIG. 1 with stent loaded.
Fig. 10 is a cross sectional view of the embodiment shown in FIG. 9 with the intermediate handle pulled toward the proximal handle.

1. A delivery catheter handle assembly comprising:
   a proximal handle attached to a proximal end of an axially elongated inner member;
   an intermediate handle attached to a proximal end of an axially elongated intermediate member, at least one of said proximal and said intermediate handles being designed to accommodate and engage the other of said proximal and said intermediate handles; and
   a distal handle attached to a proximal end of an axially elongated external member overlying at least a portion of said intermediate member.
2. The delivery catheter handle assembly according to espect 1, wherein said proximal handle and said intermediate handle include a handle-interlock mechanism, a portion of said intermediate handle being releasably retained by said proximal handle to prevent distal movement of said intermediate handle.
3. The delivery catheter handle assembly according to aspetc 2, wherein said handle-interlock mechanism is molded unitary with said proximal handle and said intermediate handle.
4. The delivery catheter handle assembly according to aspetc 1, further comprising:
   a slide-and-lock mechanism, wherein one of said proximal handle and said intermediate handle is retracted into the other to define a unitary retracted position configured to limit travel between said proximate handle and said intermediate handle.
5. The delivery catheter handle assembly according to aspetc 4, wherein said intermediate handle includes a release mechanism such that the intermediate handle can be released and positioned relatively away from said proximal handle to permit re-loading of a prosthesis.
6. The delivery catheter handle assembly according to aspetc 4, wherein said slide-and-lock mechanism is molded unitarily with said proximal handle and said intermediate handle.
7. The delivery catheter handle assembly according to aspetc 1, wherein said intermediate handle includes a mechanism at a distal position configured to prevent unintended retraction.
8. The delivery catheter handle assembly according to aspetc 1, wherein said intermediate handle includes at least one projecting finger for interlockingly connecting with said proximal handle.
9. The delivery catheter handle assembly according to aspetc 1, wherein said intermediate handle includes a gripping surface.
10. The delivery catheter handle assembly of aspetc 9, wherein said gripping surface includes wings.
11. The delivery catheter handle assembly of aspetc 9, wherein said gripping surface includes a soft grip.
12. The delivery catheter handle assembly of aspetc 1, further including a guidewire port.
13. The delivery catheter handle assembly according to 1, wherein said proximal handle includes projections to provide frictional force retaining said intermediate handle during the prosthesis loading process.
14. The delivery catheter handle assembly according to aspetc 1, wherein said proximal handle includes a mechanism for interlockingly capturing said intermediate handle during the prosthesis loading process.
15. The delivery catheter handle assembly according to aspetc 1, wherein in an as-packaged condition, the relative movement of the handles is mechanically limited.
16. The delivery catheter ofaspetc 1, wherein said intermediate handle further includes a locking mechanism for connecting with said proximal handle, said locking mechanism having a release mechanism which is recessed.
17. An apparatus for loading and delivering a prosthesis, the apparatus comprising:
   a prosthesis;
   a core segment including a proximal handle, an axially extended inner member formed integral with said proximal handle;
   a middle segment including an intermediate handle, an axially elongated intermediate member formed integral with said intermediate handle, wherein one of said proximal and said intermediate handles are designed to assume and engage other of said proximal and said intermediate handles; and
   an external segment including a distal handle, an axially elongated external member formed integral with said distal handle and overlying at least a portion of said intermediate member.
18. The apparatus according to aspetc 17, wherein said proximal handle and said intermediate handle include a handle-interlock mechanism, wherein a portion of said intermediate handle is releasably retained by said proximal handle.
19. A method for loading prosthesis onto a delivery system, the method comprising:
   providing a prosthesis delivery system including a proximal handle attached to an inner member, an intermediate handle attached to an intermediate handle, one of said proximal and said intermediate handles being designed to accommodate and engage another of said proximal and said intermediate handles, and a distal handle attached to an external member overlying at least a portion of said intermediate member, said proximal handle having a releasable locking mechanism for releasably engaging said intermediate handle;
   providing a prosthesis in a unconstrained condition;
   positioning said intermediate handle in a position away from said proximale handle and loading said prosthesis;
   moving said distal handle distally to constrain said prosthesis within said external member; and
   retracting said prosthesis basket into said axially elongated intermediate member by sliding said intermediate handle in a proximal direction until it locks into said proximal handle via said releasable locking mechanism and said basket is no longer in contact with said prosthesis.
20. The method for loading prosthesis according to aspetc 19, the method (not part of the invention) further including the steps of:
   positioning said prosthesis delivery system into a patient; and
   deploying said prosthesis by moving said distal handle in a proximal direction.
21. The method for loading prosthesis according to aspetc 20, the method (not part of the invention) further including the steps of:
   unlocking said locking mechanism and returning said intermediate handle to said distal position to reload said deployed prosthesis on said delivery system and remove or reposition said prosthesis.

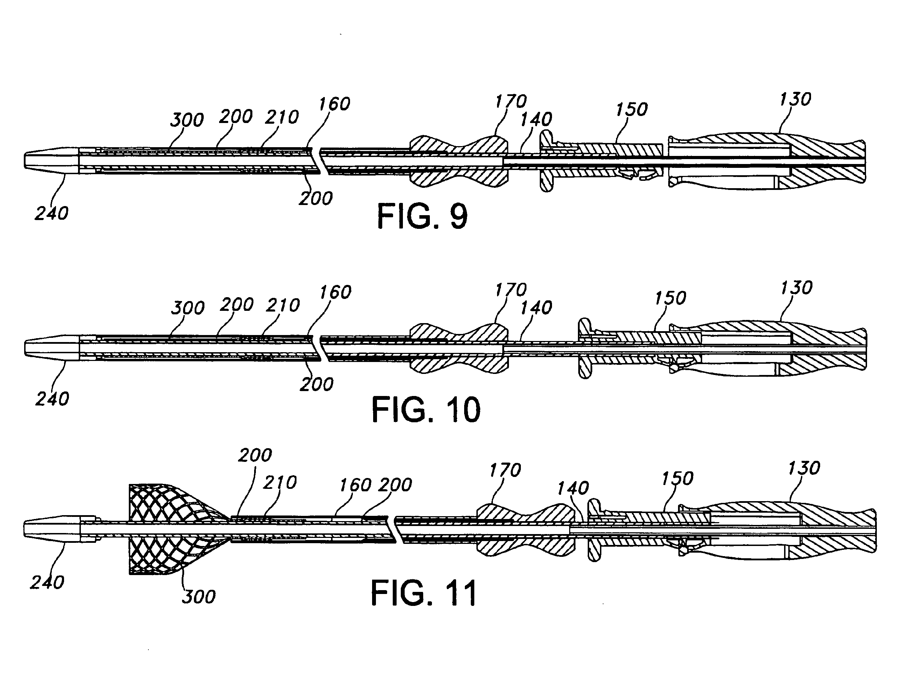

   Fig. 11 is a cross sectional view of the embodiment shown in FIG. 9 with the stent partially deployed.

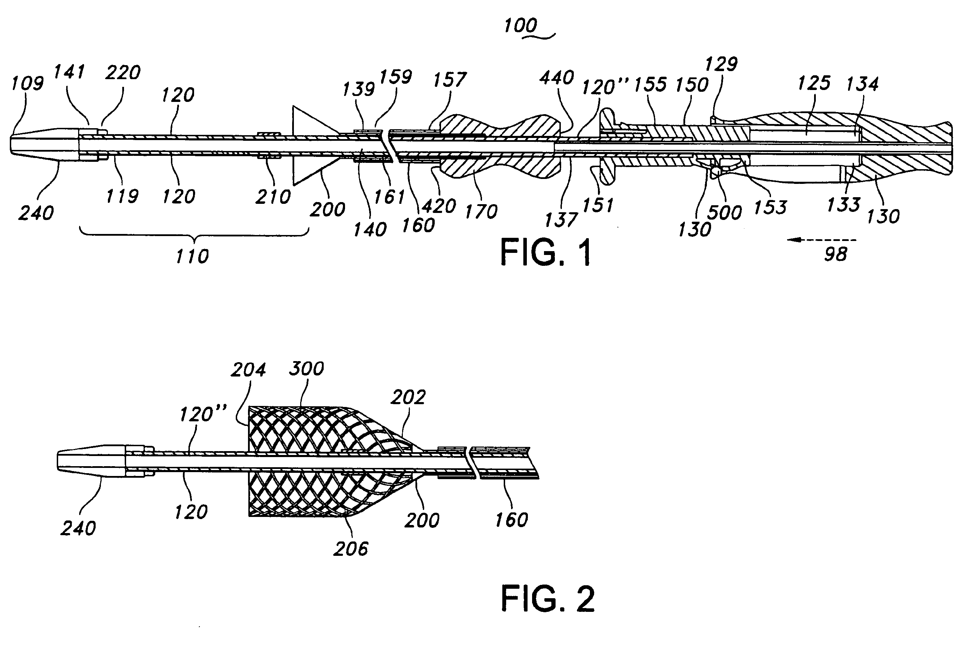

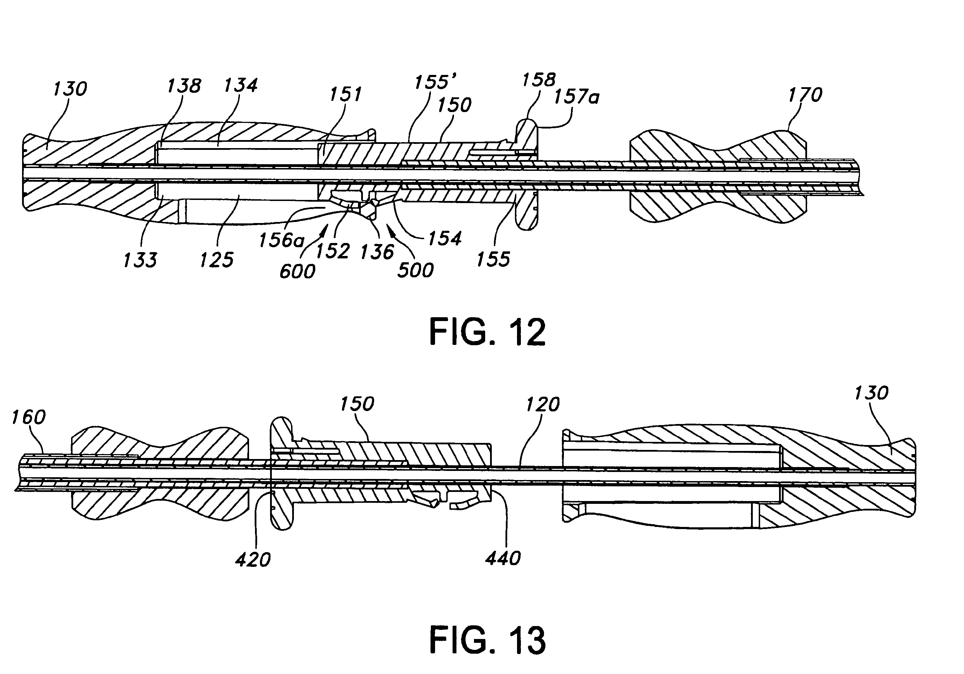

   Fig. 12 is a cross sectional view of the three handles of the embodiment shown in FIG. 1.
   Fig. 13 is a cross sectional view of the handle as shown in FIG. 12 with intermediate handle distally located.

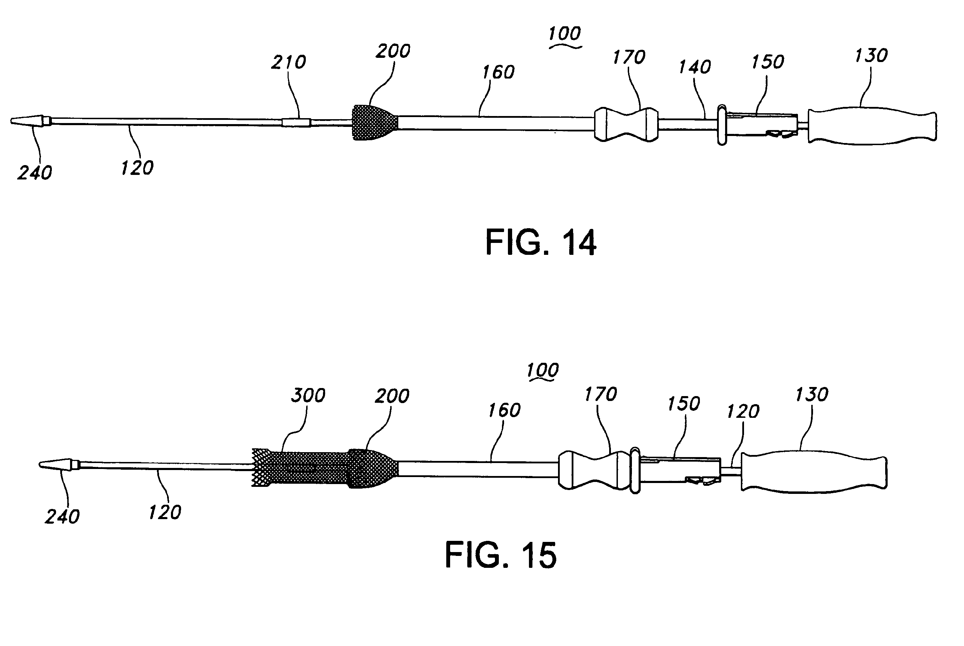

   Fig. 14 is a side plan view of the embodiment shown in FIG. 1 prior to loading of a stent.
   Fig. 15 is a side plan view of the embodiment shown in FIG. 14 with a stent partially held by a basket.

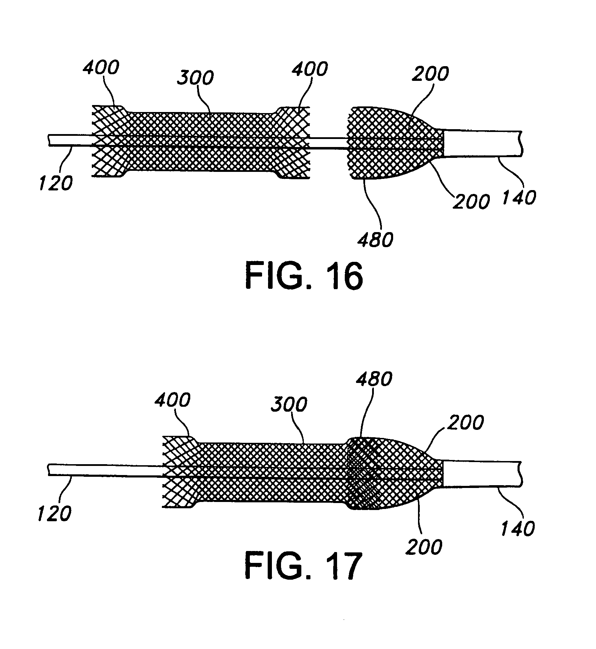

   Fig. 16 is a side plan view of the basket and the stent of the embodiment of FIG. 1.
   Fig. 17 is a side plan view of the basket and the stent of the embodiment of FIG. 1.

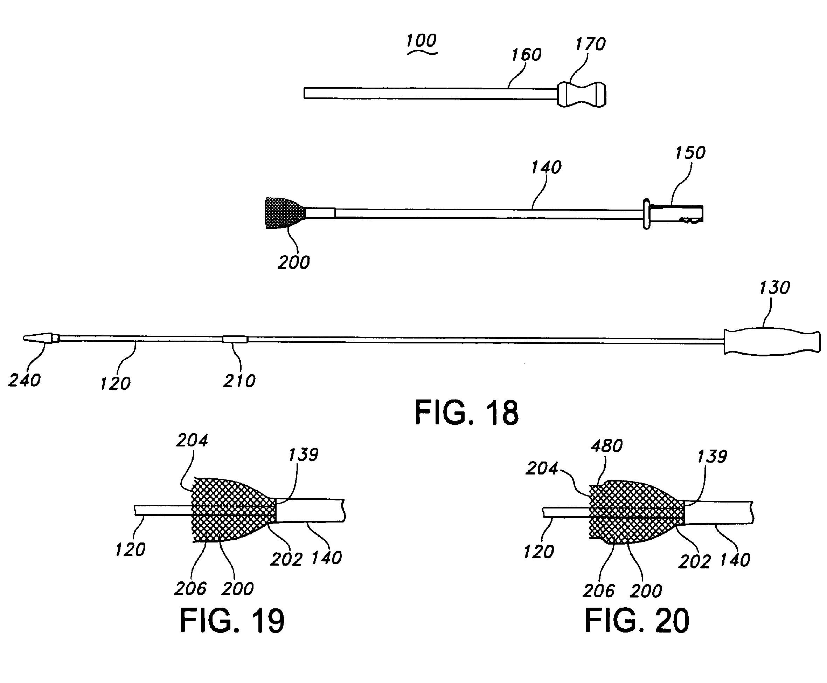

   Fig. 18 is an exploded plan view of each tubular member separate from each other.
   Fig. 19 is a side plan view of yet another embodiment of the basket as shown in FIG. 18.
   Fig. 20 is a side plan view of further yet another embodiment of the basket.

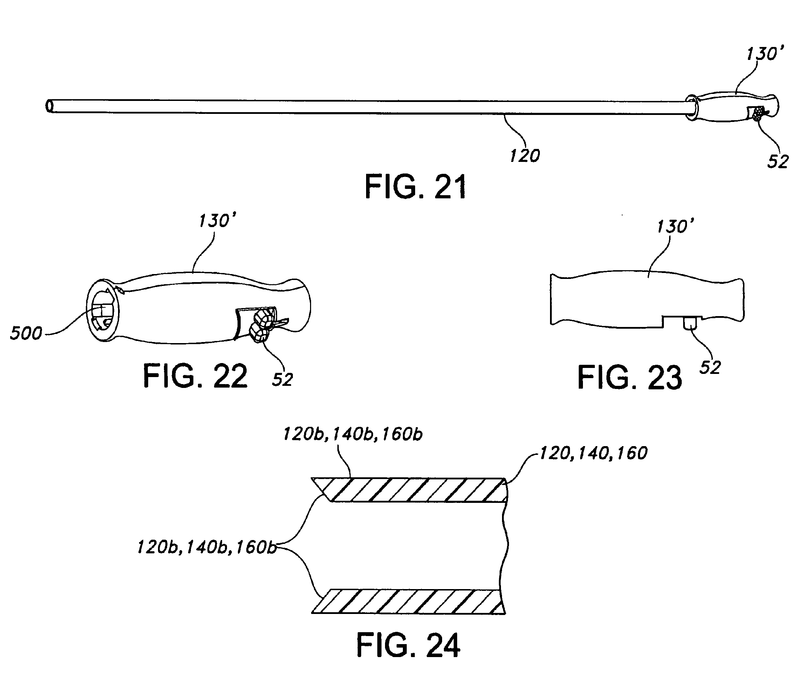

   Fig. 21 is a side perspective view of axially elongated inner member of the present invention.
   Fig. 22 is a side perspective view of the proximal handle of the present invention.
   Fig. 23 is a side plan view of the proximal handle as shown in FIG. 22.
   Fig. 24 is a cross sectional view of the bevel edge of the tubular member of the present invention.

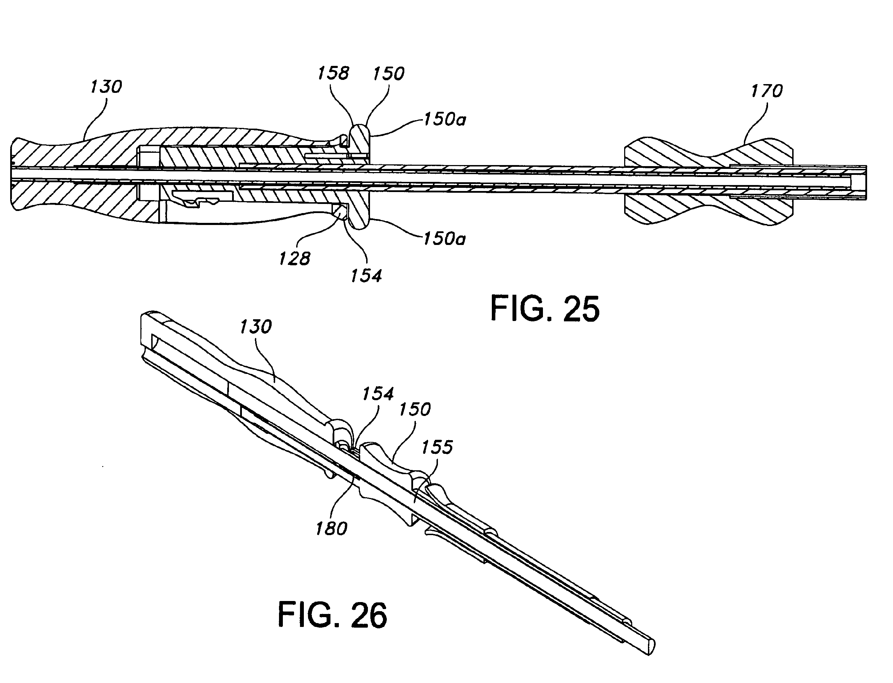

   Fig. 25 is a cross sectional view of the handles of the present invention.
   Fig. 26 is a side perspective view of the basket retractor handle of the present invention.

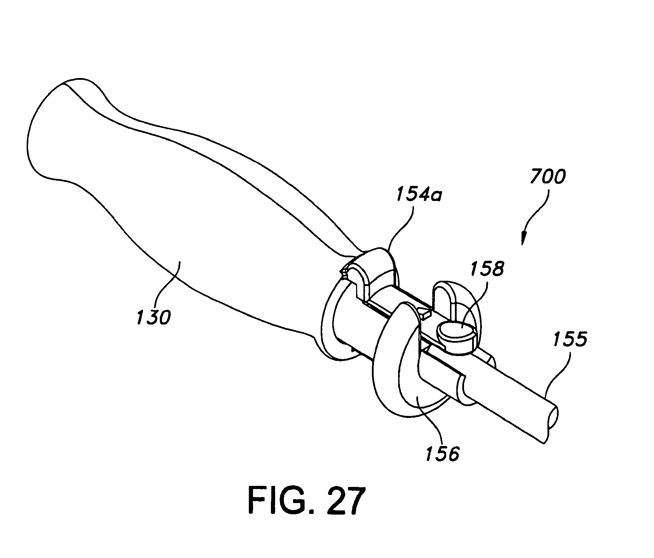

   Fig. 27 is a side perspective view of the intermediate handle release mechanism of the present invention.

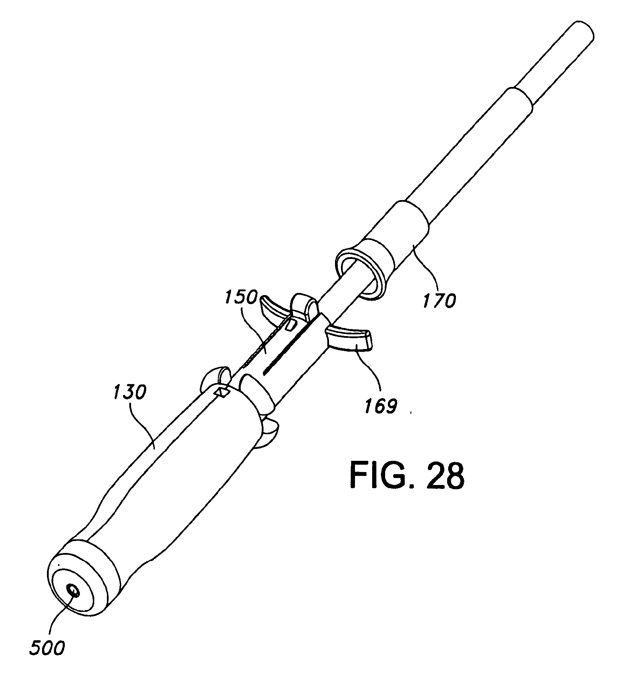

   Fig. 28 is a side perspective view of yet another embodiment of the intermediate handle with winged feature.

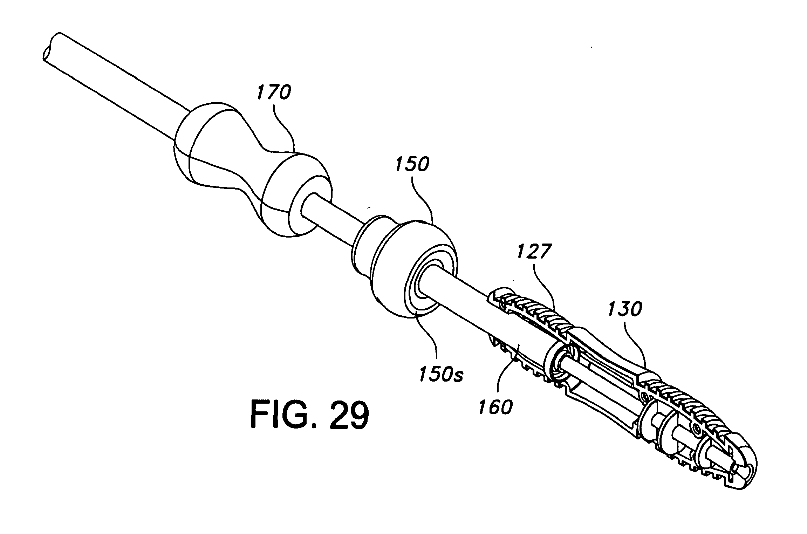

   Fig. 29 is a side cutaway view of the proximal handle with mechanisms molded therein.

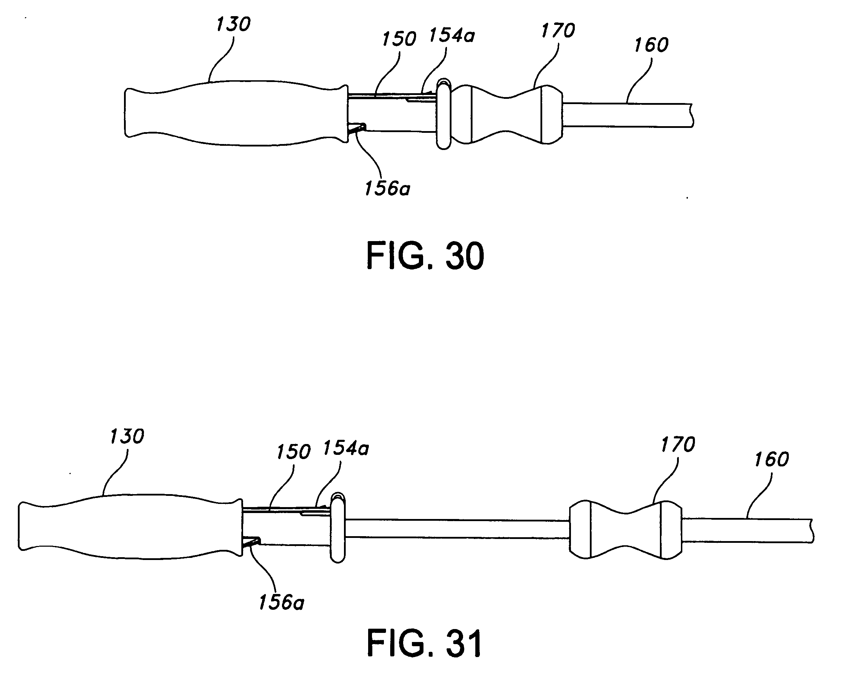

   Fig. 30 is a side plan view of the present invention with the intermediate handle at an initial position.
   Fig. 31 is a side plan view of the embodiment shown in FIG. 1 with the distal handle at a position away from the intermediate handle.

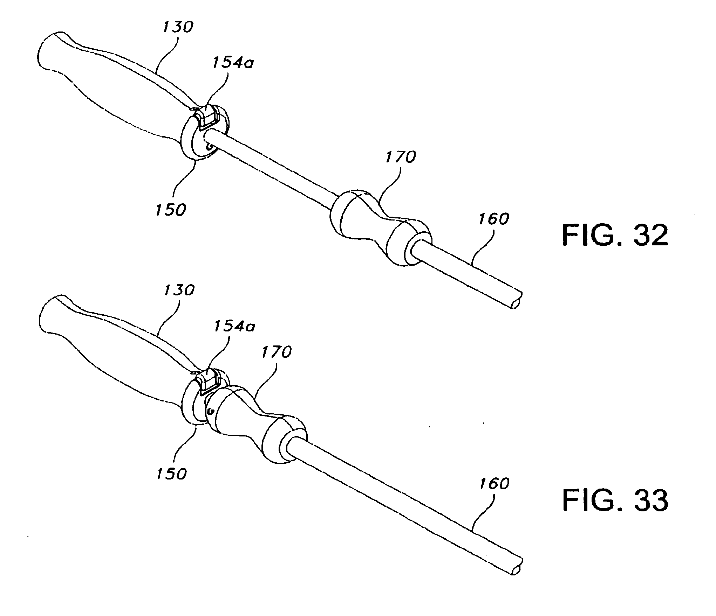

   Fig. 32 is a side perspective view of the embodiment shown in FIG. 1 with the intermediate handle fully inserted into the proximal handle.
   Fig. 33 is a side perspective view of the embodiment shown in FIG. 1 with the distal handle at a position close to the proximal handle.

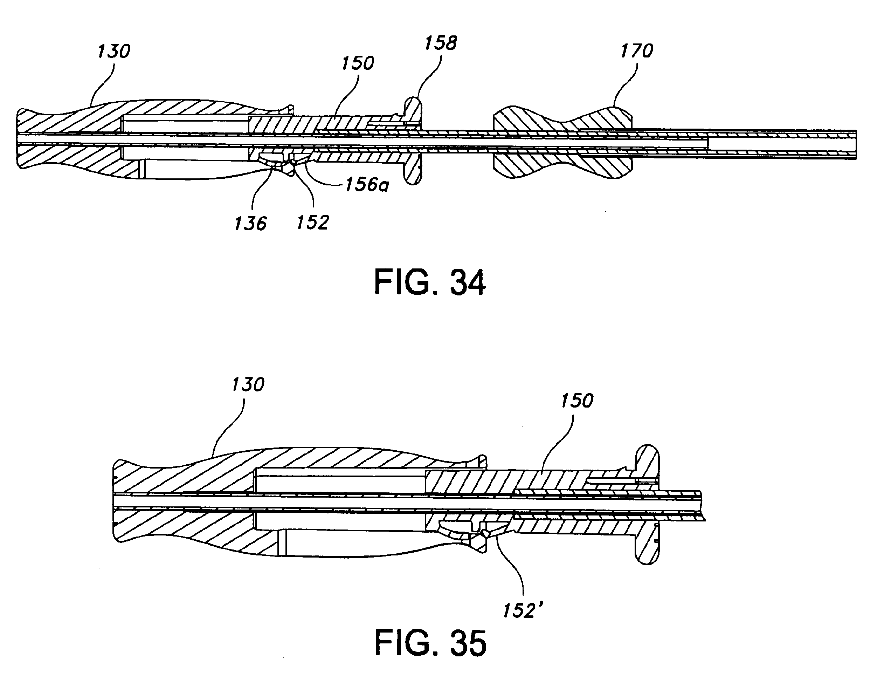

   Fig. 34 is a side cross sectional view of the three handles of the embodiment of FIG. 1.
   Fig. 35 is a side cross sectional view of yet another embodiment of the mechanism and handle interlock mechanisms.

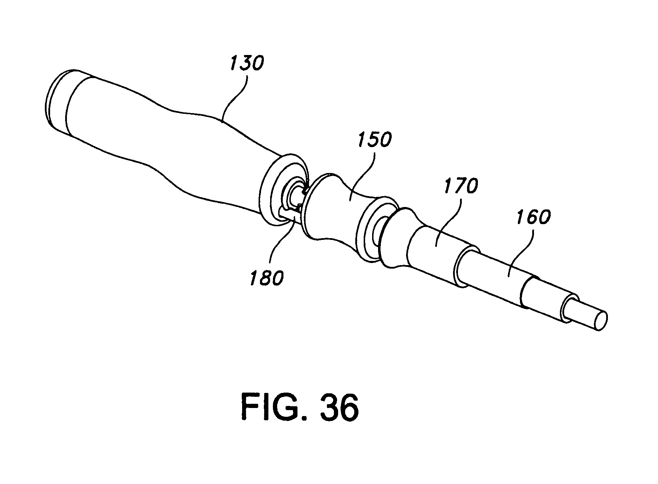

   Fig. 36 is a side perspective view of the embodiment with distal handle close to the intermediate handle.

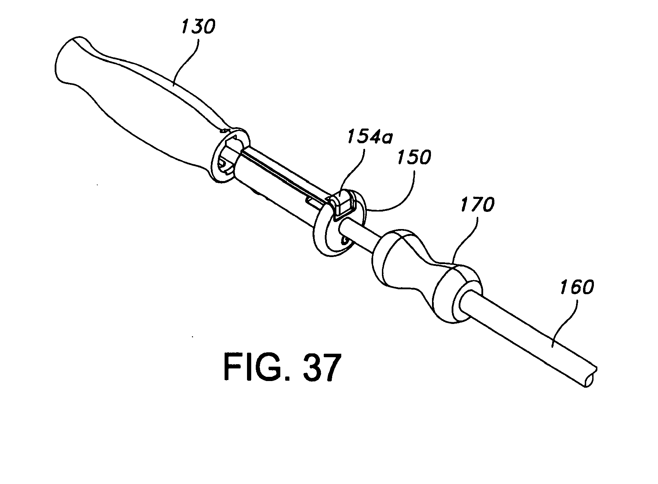

   Fig. 37 is a perspective view of the embodiment shown in FIG. 1 with intermediate handle having a button.

### DERAILED DESCRIPTION OF PREFERRED EMBODIMENT

References herein to the term "distal" and variants thereof refer to a direction away from a practitioner of the subject invention, while references to the term "proximal" and variants thereof refer to a direction towards the operator of the subject invention. Accordingly, when the terms "distal" and "proximal" are used herein in the context of an assembly device or system that is being deployed within a body, such as a human body, by an operator, the term "distal" refers to a location within or near the body that is further within the body than a location that is "proximal" to the operator.

Figure 1 is a cross-sectional view of a stent loading and delivery system **100** according to the present invention. The system **100**, as depicted, may be particularly well suited for loading, transluminal delivery and intraluminal deployment of a radially self-expanding prosthesis, such as a stent and/or a stent-graft. The system **100** may include a catheter-type device with three elongated cylindrical members concentric about an axis and having opposed proximal and distal ends. The three members can be structured as follows: A flexible axially elongated inner member **120** (which may be constructed as a tube or a solid), that may include a support platform or stent holder **210** off which a stent can be delivered; an axially elongated tubular intermediate member **140** slidably containing the inner member **120** therewithin; and an axially elongated tubular external member **160** slidably containing the intermediate member **140** therewithin, wherein all members interrelate with each other as shown in Figure 1. The members **120**, **140**, and **160** shall be described in detail below.

The members **120**, **140**, and **160** may be manipulated to cause changes to a stent deployment region **110** located at the distal end **109** of the delivery system **100**. In particular, the stent deployment region **110** may be defined as a region between the distal end **159** of the external member **160** and distal end **119** of the inner member **120** when these axial ends **159**, **119** are at a particular position farthest away from each other. In some embodiments, the stent holder **210** of the intermediate member **140** can slide to a distal position **141** past the distal end **159** of the external member **160** for receiving a stent **300**. In addition, the stent holder **210** can slide in an opposite direction toward the proximal end **157** of the external member **160** to a location **161** past the stent deployment region **110** for disengagement of the stent **300** (which is depicted in Figure 2) from the intermediate member **140**.

The mechanical relationship between the members **120**, **140**, **and 160** shall now be described. Each member of the system **100** may be defined and controlled at the proximal end by a respective handle as follows: A proximal handle **130** may be fixedly disposed at the proximal end **117** of the inner member **120**; an intermediate (for basket retraction in some embodiments) handle **150** may be disposed at the proximal end **137** of the intermediate member **140**; and a distal (for stent-deployment in some embodiments) handle **170** may be disposed at the proximal end **157** of the external member **160**. Longitudinally, the distal handle **170** may in some embodiments be disposed furthest away from the practitioner in relation to other handles or away from the proximal end **117** of the inner member **120**. The intemediate handle **150** may be disposed between the distal handle **170** and the proximal handle **130**, which may be disposed closest to the practitioner.

The handles **130**, **150**, and **170** are displaceable longitudinally along the axis **98** relative to each other thereby enabling selective deployment and retraction of the stent **300** (shown in FIG. 15). In essence, manipulation or axial movement of the handles **130**, **150** and **170** permits independent axial movement of the tubular members **120**, **140**, and **160**, respectively. For example, the intermediate handle **150** may slide between distal and proximal positions **151**, **153** so as to axially move the intermediate member **140**. Such movement may be done while keeping the other handles **130**, **170** fixed or relatively fixed to allow independent or substantially independent movement of the intermediate member **140**. While the intermediate member **140** is moved, the inner member **120** and the external member **160** may remain fixed or relatively fixed.

The present invention provides for the proximal handle **130** to cooperate with the intermediate handle **150**. Proximal handle **130** is designed to fully retract or receive a substantial portion of the intermediate handle **150**. As depicted in Figure 12, the proximal handle **130** is fabricated with an opening **132** and a receptacle **134** therein to receive a particular shaft portion **155'** of the intermediate handle **150**. Although Figure 12 may depict the opening **132** with a circular or oval shape and the receptacle **134** with a barrel shape, these shapes arc presented for illustrative purposes only. The present invention encompasses other shapes besides the illustrated shapes for these members. The shaft portion of the intermediate handle **150** may be provided with a slidable shape (tubular or otherwise) to easily facilitate sliding into the receptacle **134**. Additionally, a linear surface of the receptacle **134** may include a guide **138** and the intermediate handle **150** may include a corresponding track **156** to assist or guide the intermediate handle **150** to easily slide in and out and to prevent unwanted twisting. Alternatively, the end to end inner dimension of the receptacle **134** and the distance of the shaft **155** of the intermediate handle **150** may be fashioned with predetermined lengths. Thus shaped, the interior end **133** of the receptacle **134** may obstruct the proximal end of the intermediate **handle 150** preventing the distal end of the intermediate handle **150** from engaging or colliding into the distal end of the proximal handle **130**.

Additionally, as depicted in Figure 12, the intermediate handle **150** in combination with the proximal handle **130** may include a locking mechanism **500** (shaped, for instance, with tactile detent). The locking mechanism **500** may be configured to control the distal positioning of the intermediate handle **150** with respect to the proximal handle **130** as shall be described in detail below. Furthermore, the intermediate handle **150** in combination with the proximal handle **130** may also include a handle interlock mechanism **600** that interlocks and prohibits a complete removal of the intermediate handle **150** from the receptacle **134**. The handle interlock mechanism **600** may be shaped with tactile features, allowing the practitioner to control and lock the elements based on the practitioner's sensory perception.

The details of the mechanism **500** are described herein below. As depicted in Figure 12, a section **131** of the opening **132** may include an overhang protrusion **136** located at a distal end position **129** of the proximal handle **130**. In some embodiments, the protrusion **136** may be provided on the opposite side of the guide **138**. However, it may also be placed anywhere alongside in the interior receptacle in congruence with the portions of the intermediate handle **150**. The corresponding portions of the intermediate handle **150** may include a mechanism **154** (flexible in some embodiments) and a handle interlock **152** located at a position near the proximal end **151** of the intermediate handle **150**. The flexible mechanism **154** can be shaped with a curved sloping extension that extends toward the proximal end of the intermediate handle and bends radially inwardly when an external force is applied. When the intermediate handle **150** is retracted into the receptacle **134**, the mechanism **154** can be overcome by the protrusion **136**.

Similarly, the handle interlock mechanism **600** can be formed. In particular, the handle interlock **152** can be formed with a protrusion that extends from a location adjacent to the proximate end of the intermediate handle **150**. The shape of the handle interlock **152** can be designed with a surface region so that it will engage a region of the protrusion **136**, thereby limiting further linear movement of the intermediate handle **150**. Thus, the handle interlock **152** "captures" the intermediate handle **150**. Such mechanism **600** can also prevent the intermediate handle **150** from moving the intermediate member **140** too far distally and prevent interference of the stent loading basket **200** with the stent holder **210**. In some embodiments, the handle interlock **152**, as well as the mechanism **154**, may be provided on the proximal underside of the intermediate handle **150**. However, similar to the placement of the protrusion **136**, these mechanisms may be placed anywhere alongside the shaft **155** of the intermediate handle **150** in congruence with the location of protrusion **136**.

The locking mechanism **500** and the handle interlock mechanism **600** can be enhanced by another mechanism for a stable state. Also seen in Figure 12, a ledge **156a** may be provided between the mechanism **154** and the handle interlock **152** so as to provide a recessed stable region for the protrusion **136**. Thus formed, as the intermediate handle **150** protracts from a fully retracted position, the protrusion **136** may slide over the mechanism **154** and come to rest on the ledge **156a**. Once the protrusion **136** rests on the ledge **156a**, the handle interlock **152** catches and prevents the protrusion **136** from sliding further toward the proximal end **151** of the intermediate handle **150** and prevents the intermediate handle **150** from being dislodged completely. The position where the protrusion **136** rests on the ledge **156** defines the position where the two handles **130** and **150** become locked together and the positions of the members **120** and **140** are stationary relative to each other. This position also defines the initial stent loading position where the practitioner can easily place a stent for loading. This position allows the practitioner to load the stent, prior to completely retracting the intermediate handle **150** into the proximal handle **130**. Therefore, the protrusion **136** in combination with the handle interlock **152** and the mechanism **154** provide a systematic control that not only stabilizes the position of the intermediate member **140**, but also allows for proper stent placement. Thus shaped, the relative position and movement of the proximal and intermediate handles **130**, **150** can be mechanically limited by the enclosed and bounded design of the handles **130**, **150** and **170**.

The protrusion **136**, the handle interlock **152** and the mechanism **154** combination may be further enhanced in several different ways. For instance, the intermediate handle **150** includes a distal flange portion **157a t**hat serves as an alternative stop when the intermediate handle **150** is inserted into the receptacle **134**. Furthermore, the flange portion **157a** may be designed to slide neatly into a distal recessed region **125** on the proximal handle **130**. Thus, the intermediate handle **150** can be flush within the proximal handle **130** when retracted, or can be designed to be less accessible as shown in Figure 27. When the release mechanism **700** for the intermediate handle **150** is less accessible to the practitioner, it is less likely that the stent may be prematurely deployed.

In addition, the flange portion **156a** of the intermediate handle **150** may also include a release mechanism such as a button **158** that protracts and retracts the intermediate handle **150** in relation to the proximal handle **130**. In particular, the release mechanism or button **158** actuates the depression of mechanism **154a** (which may function as a detent) and disengages the intermediate handle **150** from the protrusion **136**, thereby facilitating a full retraction into the proximal handle **130** when the stent needs to be loaded. Figure 37 is a partial perspective view of the delivery system **100** showing the handles **130**, **150**, **170** and mechanism **154a**, inter-related as shown. Figure 26 depicts the details of the opening **132** and a substantial shaft portion **155** of the intermediate handle **150** immediately prior to being retracted into the receptacle **134**. As depicted in Figure 25, the intermediate handle **150** may also include the release mechanism **158** on the top portion **150a** to actuate the movement of the mechanism **154** on the bottom portion **150b** of the intermediate handle **150**, thereby allowing the intermediate handle to fully retract into the proximal handle **130**. In addition, the proximal handle **130** may include a flange portion **128** to cooperate with the release mechanism **158**. Thus, the release mechanism **158** of the intermediate handle **150** can be designed to be flush with the flange portion **128** of the proximal handle **130** when retracted within the receptacle **134**, or can be designed to be less accessible as shown in Figure 27.

Several other features are envisioned by this invention. For instance, in an alternate form embodiment, the handle interlock **152** and mechanism **154** can be a single molded feature in the design of the intermediate handle **150**, as depicted in Figure 12. In some embodiments, as illustrated in Figure 28, the intermediate handle **150** can have alternate features. For example, the intermediate handle **150** may include winged projections **169**, in some embodiments at its distal end **153** to improve grip. The winged projections **169** may allow a practitioner to more easily grasp one of the projections **169** by utilizing his/her fingers or any other means. The intermediate handle **150** can have other alternate features to improve grip as well. For instance, in yet another embodiment of the delivery system **100** according to the current invention includes the position of the intermediate handle **150** being controlled by another controlling means such as a rod (not shown), that interlocks with the proximal handle **130**. In some embodiments, the position of the intermediate handle **150** can be controlled by another grip such as a rod that interlocks with the proximal handle **130**. Also, the retention of the intermediate handle **150** can be made permanent by features such that the handles cannot be separated if this feature is desirable. For instance, the handles may interlock with screw threads, ratchets, adhesives, friction, or other mechanisms as will be understood by a person of ordinary skill in the art.

Additionally, each grip portion of the handles **130**, **150**, and **170** may also include additional inventive features. For instance, by molding ribs **127** onto the proximal handle **130** as depicted in Figure 29, the proximal handle **130** can be designed to engage the intermediate handle **150** during the assembly process to improve attachment of the catheter tubular members. As depicted in Figure 29, in some embodiments, the intermediate handle **150** may include a flange style or a knob-style stop **150s** with a mechanical feature which correspondingly interacts with the proximal handle **130**. Furthermore, the mechanism **154** for intermediate handle **150** can also be molded into the handle interior of the proximal handle **130** as depicted in Figure 29. Thus shaped, the catheter's tube may pass through and can be secured in the ribs **127** of the proximal handle **130**. In some embodiments of this invention, the proximal handle **130** may be designed to better grip and hold the intermediate handle **150** during the assembly process. Also, all handles may have softer polymers for grip or ergonomic designs for surface designs. For instance, molding ribs **127** may be fashioned onto the tubular members of the proximal handle **130** to improve securement into the intermediate handle **150**. In addition, all mechanism may be molded in place. For instance, in some embodiments, mechanisms **154** for the intermediate handle **150** may also be molded into the interior of the handle **150**.

As depicted in Figure 1, the system **100** may further include a distal tip **240** disposed at the distal end of the inner member **120**. Thus, the inner member **120** may be in some embodiments defined at one end by the distal tip **240** and at the other end by the proximal handle **130**. Distal tip **240** may be symmetrically optimized for atraumatic insertion and withdrawal of system **100**, for example as a tapered tip. Also, the distal tip **240** may comprise soft materials. Thus formed, distal tip **240** may be useful for navigating bodily lumens without causing trauma to the same.

In addition, any of the members may have a varying stiffness at any portion along its length to improve the performance of system **100**. For instance, the inner member **120** may be comprised of any variety of physical materials that can flex according to the practitioner's needs. In some embodiments, the inner member **120** may have a plastic (synthetic) or a polymer (which may even include natural materials) core. Also, the proximal end **117** of the inner member **120** may comprise a relatively stiff portion **120"** in relation to the distal portion **119**. The stiff portion **120"** of the inner member **120** may secure to the proximale handle **130**.

Any of the members may have any number of varying diameters along its length provided that the variations serve to improve control of stent deployment of loading. For instance, the inner member **120** may include a distal flexible thick portion **120'** extending from and having a larger diameter than the proximal portion **120"** such that the proximal portion **120"** may be slidably disposed within the intermediate handle **150** while the distal portion **120'** may be slidable through only a portion of the intermediate handle **150**. In such a case, the intermediate handle **150** may have a distal opening **420** larger than a proximal opening **440**. Such arrangement may function as a stop or limit for the axial movement of the distal portion **120'** relative to the intermediate member **140** during the loading of the stent **300.** The distal portion **120'** may be relatively flexible radially. This may provide limited flexibility so that the catheter system **100** can readily be pushed from the handle **130** through the patient's vessel with little risk of kinking. The flexible distal portion **120'** may be configured to support a compacted stent within the external member **160** proximate the distal end tip **240** for deployment from the system **100** in an expanded form within a patient's vessel.

The movement between members may be improved by reducing surface area contact, for example by having raised areas upon which an outer member would contact an inner member. Such raised areas or reduced surface areas may include bumps, ribs, etc.

The tubular members **120, 140**, and **160** may be formed of a body compatible material such as a biocompatible polymer. In some embodiments, the stiff portion **120"** of the core inner member **120**, which extends along a substantial length of the catheter system **100**, may be formed of a plastic material such as PEX (cross-linked polyethylene) or an elongated coiled spring formed of plastic or metal such as Nitinol. In addition, the external member **160**, which functions as a slippery outer sheath, may in some embodiments be formed of a radially flexible (bend sideways), axially stiff (not stretchable lengthwise) material such as polytetrafluorethylene (PTFE) or other like material as shall be described below.

The present invention, however, is not limited to the use of just PTFE as the external member, and other materials, including combinations of materials, may suitably be used. Specifically, any of the materials may be reinforced or improved by addition of reinforcement materials such as braids, coils or the like and/or geometric features and/or localized addition or subtraction of materials. For example, non-limiting examples of suitable biocompatible polymers also include, and are not limited to, polyolefins such as polyethylene (PE), high density polyethylene (HDPE) and polypropylene (PP), polyolefin copolymers and terpolymers, polyethylene terephthalate (PET), polyesters, polyamides, polyurethanes, polyurethaneureas, polypropylene and, polycarbonates, polyvinyl acetate, thermoplastic elastomers including polyether-polyester block copolymers and polyamide/polyether/polyesters elastomers, polyvinyl chloride, polystyrene, polyacrylate, polymethacrylate, polyacrylonitrile, polyacrylamide, silicone resins, combinations and copolymers thereof, and the like. Materials for the tubular members **120**, **140**, **160** may be same or different.

The tubular members **120**, **140**, and **160** may also have a surface treatment and/or coating on their inner surface, outer surface or portions thereof. A coating need not be applied to all of the tubular members **120**, **140**, **160**, and individual members may be coated, uncoated, partially coated, and the like. Useful coating materials may include any suitable biocompatible coating. Non-limiting examples of suitable coatings include polytetrafluoroethylene, silicone, hydrophilic materials, hydrogels, and the like. Useful hydrophilic coating materials include, but are not limited to, alkylene glycols, alkoxy polyalkylene glycols such as methoxypolyethylene oxide, polyoxyalkylene glycols such as polyethylene oxide, polyethylene oxide/polypropylene oxide copolymers, polyalkylene oxide-modified polydimethylsiloxanes, polyphosphazenes, poly(2-ethyl-2-oxazoline), homopolymers and copolymers of (meth) acrylic acid, poly(acrylic acid), copolymers of maleic anhydride including copolymers of methylvinyl ether and maleic acid, pyrrolidones including poly(vinylpyrrolidone) homopolymers and copolymers of vinyl pyrrolidone, poly(vinylsulfonic acid), acryl amides including poly(N-alkylacrylamide), poly(vinyl alcohol), poly(ethyleneimine), polyamides, poly(carboxylic acids), methyl cellulose, carboxymethylcellulose, hydroxypropyl cellulose, polyvinylsulfonic acid, water soluble nylons, heparin, dextran, modified dextran, hydroxylated chitin, chondroitin sulphate, lecithin, hyaluranon, combinations and copolymers thereof, and the like. Non-limiting examples of suitable hydrogel coatings include polyethylene oxide and its copolymers, polyvinylpyrrolidone and its derivatives; hydroxyethylacrylates or hydroxyethyl(meth)acrylates; polyacrylic acids; polyacrylamides; polyethylene maleic anhydride, combinations and copolymers thereof, and the like. Additional details of suitable coating materials and methods of coating medical devices with the same among other features may be found in U.S. Patent Nos. 6,447,835 and 6,890,348. Such coatings and/or surface treatment can be desirably disposed on the inside or a portion thereof of the external member **160** to aid, if desired, in loading and/or deploying of the stent.

Referring back to Figure 1, the distal end tip **240** of the catheter system **100** may be fabricated to provide sideways mobility. That is, the distal end tip **240** remains free to float in radial (sideways) directions different from the axial direction of the core inner member **120**. Thus, even a slight force directed upon the distal end tip **240** may nudge its position away from the source of the applied force. This feature can ease the practitioner's task in navigating the catheter system **100** through severely tortuous paths often associated with procedures in a patient's body, reducing steps and time necessary for delivering a stent to a delivery site.

Further as depicted in Figures 19 and 20, in some embodiments, the system **100** may include a stent basket **200** having opposed proximal and distal ends **202**, **204** for engaging a stent **300**. For instance, the proximal engaging end **202** may be securely disposed to or provided at the distal end **139** of the stent-loading intermediate member **140**. The stent basket **200** may have a truncated-conical shape, being smaller at its proximal end, i.e., outwardly diverging in a distal direction from its proximal engaging end. The stent basket **200** may be a thin film which can collapse such that the stent basket **200** may be slidably contained within the distal end of the external member **160**. Alternatively, the stent basket **200** may include a radially distensible member **200** as depicted in Figure 20 which can be collapsible such that the stent basket **200** can be slidably contained within the external member **160**. For instance, the stent basket may be a porous tube, a flexible tube, or any other configurable tube. In some embodiments, the stent basket **200** may be a polymeric member **200**. The stent basket **200** may include, in part or substantially, braided filaments **206**. The braided filaments **206** may include polymeric filaments, metallic filaments and any other suitable filaments. Alternatively, the braided filaments may be contained within a thin polymeric film.

Another preferred feature of the present invention is that a stent holder **210** can be provided on a distal portion **119** of the inner member **120** to temporarily hold the stent in place without any substantial external force acting on it. The stent holder **210** may be further defined by a tubular band **220.** In some embodiments, the stent holder **210** may releasably hold stent **300** within system **100** even after the stent basket **200** may be axially displaced away from the stent **300**. Such feature may allow, if desired, for a large portion of the stent **300** to be deployed and then be recaptured or re-engaged by stent basket **200** prior to complete deployment of the stent **300.** The recapturing may be achieved by axially sliding the external member **160** over the stent **300.** Moreover, the stent basket **200** may be repositioned between the inner member **120** and the external member **160,** for example, by axially advancing the stent basket **200** to reposition the stent **300** therein between. Furthermore, the whole system **100** may be moved proximally or distally to reposition the stent **300** therein.

These features may provide, among other things, reloading ability (reconstrainability) of the stent **300** within the system **100** of the present invention. For example, the external member **160** may be advanced over the stent **300** to a location distally past the tubular band **220** to releasably and securely set the position of the stent basket **200** and/or the intermediate member **140** relative to the position of the inner member **120.** The external member **160** may be retracted proximally past the tubular band **220,** thereby allowing repositioning of the stent **300** within external member **160** and/or over the inner member **120.** The external member **160** may be re-advanced over the stent **300** and the tubular band **220** to releasably and securely reset the position of the stent basket **200** and/or the intermediate member **140** relative to the position of the inner member **120,** thereby reloading the stent.

The discrete members **120, 140,** and **160** of the catheter system **100** facilitate flexibility and control. For instance, while the proximal and intermediate handles **130, 150** are fixed or relatively fixed, the practitioner may axially move distal handle **170** to cause the distal end **159** of the external member **160** to slide substantially up to the distal end **119** of the inner member **120.** The independent or substantially independent sliding movement of the external member **160** is independent or substantially independent of the inner member **120** and the intermediate member **140** remain fixed or relatively fixed (for instance, to hold the stent).

In some embodiments, all three members, **120, 140** and **160** may be interlocked for initial deployment, repositioning and removal. Optionally, the proximal handle **130** may be axially moved between distal and proximal positions, while keeping the other handles **170, 150** fixed or relatively fixed. This axial movement facilitates independent or substantially independent movement of the inner member **120** while the external member **160** and the intermediate member **140** remain fixed or relatively fixed.

The present invention is not limited to those movements. In essence, the invention provides for axial movement of one particular member while the two other members are fixed relative to each other. Thus, the present invention includes any combined movement of the members **120, 140,** and **160.** For instance, any two of the handles **130, 150** and **170** may be moved or manipulated in concert as a pair while keeping the third or non-paired handle fixed or relatively fixed. This manipulation by the practitioner allows concurrent movement of two tubular members while keeping the third tubular member fixed or relatively fixed.

For example, the external member **160** and the intermediate member **140** may be moved in concert while keeping the inner member **120** fixed or relatively fixed. This movement can be achieved when the practitioner manipulates the distal handle **170** and the intermediate handle **150** in concert (in contrast to the movement of the proximal handle **130** above), while keeping the proximal handle **130** fixed or relatively fixed. Alternatively, the external member **160** and the inner member **120** may be moved in concert while the intermediate member **140** may be kept fixed or relatively fixed. This movement can be achieved by manipulating the distal handle **170** and the proximal handle **130** in concert while keeping the intermediate handle **150** fixed or relatively fixed. Moreover, the inner member **120** and the intermediate member **140** may be moved in concert while keeping the external member **160** fixed or relatively fixed. Similarly, this movement can be achieved by manipulating the proximal handle **130** and in concert with the intermediate handle **150** while the distal handle **170** is kept fixed or relatively fixed. It should be noted that a similar effect may be achieved by manipulating the distal handle **170** while the proximal handle **130** and the intermediate handle **150** are kept fixed or relatively fixed.

As described above, the stent basket **200** may be useful for engaging and securing a proximal end of a stent **300** and compressingly loading the stent **300** into the system **100** through axial manipulation. Such loading may be accomplished by gliding the intermediate handle **150,** against the external member **160** and kept in that position until the desired stent delivery location within a bodily lumen (not shown) is reached. Figure 1 also shows a stent holder **210** disposed on the inner member **120** at a distal location away from the stent basket **200.** The system **100** may be configured to provide this stent holder **210** at the distal location when the intermediate handle **150** is proximally placed away from the distal handle **170.** The system **100** may further advantageously include a tubular band **220** disposed toward the distal end of the inner member **120.** Such tubular band **220** may releasably secure the stent **300** in the stent deployment region **110** between the inner and external members **120, 160.**

Another feature of the present invention is that the stent holder **210** may be distally spaced apart from the stent basket **200.** Such axial displacement (away from the stent basket **200**) allows the stent holder **210** to releasably hold the stent **300** within the system **100** even after the stent basket **200** is withdrawn from the stent **300.** Such feature facilitates, if desired, for a large portion of the stent **300** to be deployed and then be recaptured by the system **100** prior to complete deployment of the stent **300.** Such recapturing or reconstrainability may be achieved with the filament described below or by axially sliding the external member **160** over the stent **300.** Moreover, the stent basket **200** may be repositioned within the inner member **120** and the external member **160.** For instance, the practitioner may axially advance the member **200** to reposition the stent **300** therein between. Furthermore, the whole system **100** may be moved proximally or (distally to reposition the stent **300** therein in a body lumen.

Although the present invention is not so limited, the reconstrainability feature of the invention can be configured as follows. A practitioner may advance the external member **160** over the stent **300** to a location distally past the tubular band **220.** The external member **160** advancement over the stent **300** releasably secures the stent basket **200** and/or the intermediate member **140** relative to the position of the inner member **120** to hold the stent **300** in place. When the stent is placed in the body lumen, the external member **160** may be retracted proximally past the tubular band **220,** thereby allowing repositioning of the stent **300** within the external member **160** and/or over the inner member **120.** If the practitioner decides to deploy the stent at a different location, the practitioner may re-advance the external member **160** over the stent **300** and the tubular band **220.** This re-constraining releasably and securely reset the position of the stent basket **200** and/or the intermediate member **140** relative to the position of the inner member **120.**

Although the current invention is not so limited, and the system **100** may be suited for delivery of many intraluminary devices, it may be particularly useful in loading and releasing the stent **300** such as the embodiment shown in Figure 2. Figure 2 illustrates a radially self-expanding stent **300** that can be radially compressed and loaded into system **100.** This stent **300** may be transluminally delivered to an intended intraluminal treatment site, then released from the system for radial self-expansion against surrounding tissue of a bodily lumen. The degree of elongation may depend upon the structure and materials of the stent **300** and may be quite varied. The diameter of the stent **300** also may become several times smaller as it elongates. The radially distensible stent **300** may be a polymeric stent, including a braided stent. A graft, such as a covering, a liner, a film, a coating and combinations thereof, may be disposed over at least a portion of the stent. In some embodiments, the stent **300** may be a braided polymeric stent and the graft may be a silicone coating or film.

Figure 2 also depicts the stent basket **200** details for the system **100** of the present invention. As depicted, the handles **150** and **170** can be disposed relatively towards one another such that the stent basket **200** may be exposed. When the stent basket **200** is exposed, it may include a distal portion radially extended to a diameter, for example, substantially larger by at least about double the diameter, than the outside diameter of the external member **160.** The stent basket **200,** which is depicted as being in the shape of a funnel or a basket, may be bonded, crimped or otherwise secured to the distal end of the intermediate member **140.** In some embodiments, the stent basket **200** may have a truncated-conical shape, outwardly diverging in the distal direction from its proximal end, e.g., the proximal end being smaller than the distal end. The proximal end of the stent basket **200** may have a diameter equal or substantially equal, including slightly larger, to the diameter of the intermediate member **140,** but less than the diameter of the external member **160.**

The stent basket **200** may be formed of a thin polymeric film, for example, but not limited to, polyamide, such as polyamide 6-6 or nylon, PET or PTFE. In some embodiments, the film may be compliant, so that the funnel can alternatively assume an open configuration as seen in Figure 1 for receiving a proximal end of stent **300.** The film may also assume a collapsed configuration to allow the stent basket **200** to be accommodated or contained within external member **160.** In some embodiments, the stent basket **200** may be resilient and can assume the open configuration in the relaxed state when free of external stresses. Alternatively, the stent basket **200** may be pliable, in particular radially distensible, mesh, weave or braid. The stent basket **200** may be of any reasonable length and/or diameter to permit the loading of the stent **300.** The stent basket **200** may have a beveled edge or profile for easier loading, removing or repositioning of the stent **300.** Further, the stent basket **200** may only partially circumferentially surround or encompass the intermediate member **140.** Still further, the stent basket **200** may be split or slit at either or both of its distal and proximal ends. Moreover, the stent basket **200** may comprise a film with pores, thin spots or cut-outs.

As depicted in Figure 13, in another embodiment, the distal and proximal openings **420, 440** of the intermediate handle **150** may be the same, substantially the same or about the same. In such embodiments, the intermediate handle **150** may be temporarily held against or near the distal handle **150** during loading of the stent **300** into the system **100.**

Figures 14 and 15 depict top planar views of the system **100** of the present invention. In this particular embodiment, the stent basket **200** may be a radially distensible basket, which can be made of similar materials or different materials from the material of the stent **300.** As depicted in Figures 16 and 17, the stent basket **200** may have a truncated-conical shape **460,** outwardly diverging in the distal direction from its proximal end. This shape may then merge, desirably seamlessly, into a straight or substantially straight cylindrical portion or rim portion **480.** The stent basket **200** may be radially distensible, i.e., it may assume an enlarged state when released from a contracted state, such as being compressed within the external member **160.** The stent basket **200** can be especially useful for engaging the stent 300 having an outwardly extending end.

As depicted in Figure 19, the stent basket **200** may be made radially distensible with a truncated-conical shape by compressing a proximal portion of cylindrical stent basket **200** onto the intermediate member **140.** Additionally, the rim portion **480** of the stent basket **200** may be inwardly biased, as depicted in Figure 20. Such alternate stent engaging designs can be useful with the different stent configurations described herein. In some embodiments, the stent basket **200** may be secured to intermediate member as well.

Figure 18 is a top planar view of the different elements of the system **100** of the present invention in an "unassembled" stage. The inner member **120** can be the longest member. The intermediate member **140** may be smaller than the inner member **120,** but longer than the external member **160.** Finally the external member **160** may typically be the shortest of the members. The present invention, however, is not so limited and other tube length configurations may suitably be selected.

In the alternative, a central lumen can extend through the catheter system **100** and receive a guide wire. With the central lumen, the practitioner can first position the guide wire in the patient and then slide the catheter system **100** over the guide wire to position the catheter system **100** within the patient's vessels with relative ease. Moreover, the inner member **120** may be modified to enhance repositioning and/or retrieval of the stent **300.** For example, as depicted in Figures 21 to 23, the proximal handle **130'** may include prongs **52.** Prongs **52** are useful for securing a filament (not shown) to the outside of the handle **130'.** The filament (not shown) may then be disposed within the cavity or lumen **500** of the handle **130.** The filament may then exit at an intermediate point whereby the filament may be secured to the stent **300.** The filament may be manipulated by the practitioner to reposition the stent **300** during or after its delivery. Upon completion of the stent delivery, the filament may be removed, for example by cutting, from the stent **300.**

Such additional features are further described in U.S. Application No. 11/437,455, entitled "Apparatus and Method for Loading and Delivering a Stent" (U.S. Pub. No. 2007/0270937 A1), filed on May 19, 2006, attorney docket 792-36, U.S. Application No. 11/437,459, entitled "Apparatus and Method for Loading and Delivering a Stent Using a Suture Retaining Mechanism" (U.S. Pub. No. 2007/0270931 A1), filed on May 19, 2006, attorney docket 792-44, and, U.S. Application No. 11/437,889, entitled "Apparatus and Method for Loading and Delivering a Stent" (U.S. Pub. No. 2007/0270932 A1), filed on May 19, 2006, attorney docket 792-57.

Further, the tubular members **120, 140,** and **160,** may have a beveled or slanted edge at their distal end, proximal end or combinations thereof. In some embodiments, such beveled edges may have smooth or round edges and accordingly may include rounded or smoothly contoured portions. For instance, as depicted in Figure 24, tubular members **120, 140, 160,** may have an inwardly beveled edge **120a, 140a, 160a** at their respective distal ends **120b, 140b, 160b.** In some embodiments, the beveled edge **160a** can be an inwardly beveled edge on the distal end **160b** of the external member **160.** Such beveled edges, in particular, beveled edge **160a,** are useful in aiding the loading and/or deployment of the stent **300.** As depicted in Figure 24, an inwardly beveled edge or end can be defined as a location where the wall of the tubular member has a greater longitudinal expanse at its outer wall portion as compared to its inner wall portion. In other embodiments, the edge may be rounded as well.

In addition, in some embodiments, the intermediate member **140** including the intermediate handle **150,** and optionally the stent basket **200,** may be releasably disposed to be completely removable from the device or system **100.** For example, after loading the stent **300** into the system **100,** the intermediate member **140** may be pulled proximally and completely detached from between the inner and external members **120, 160.** Thus, although other methods exist, the intermediate member **140** may be split and pulled away from the inner member **120.**

Another aspect of the invention improves the fluoroscopic observation of the distal end of system **100.** Specifically, the distal end tip **240** can be formed of a radiopaque material or provided with a radiopaque marker. In addition, the core tubular member **120** and/or the extreme distal end of the tubular member **140** can also be provided with indicia material/marker and may be MRI compatible or of a type only visually perceived (e.g. colored baskets). Further the material of the system may be metal free as well.

Thus, the stent delivery system **100** of this invention can enable a practitioner to position a compacted stent at a selected location with full control. The practitioner may, with optional aid of selected indicia colors and interlocking basket **200,** partially deploy the stent. Then, the practitioner may, upon observing a problem such as incorrect positioning or the like, either fully deploy the stent **300** or return the stent **300** to its compacted form within the basket **200.**

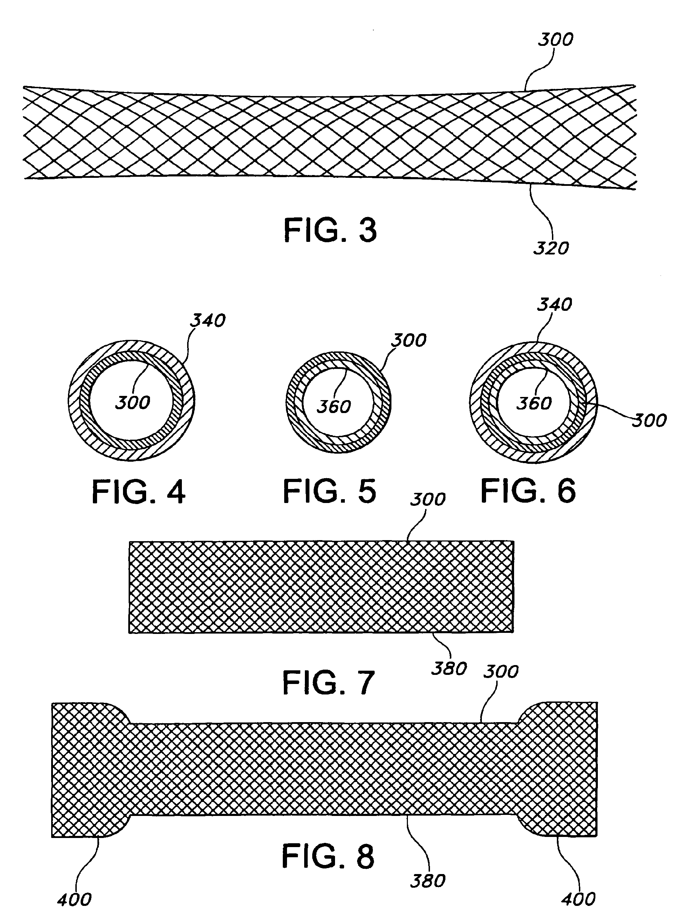

Any stent may be used in accordance with the present invention, and the invention can be constructed to accommodate stents of various sizes and configurations. For example, a radially distensible stent which does not substantially longitudinally elongate upon radial contraction is also useful. A non-limiting example of such a stent may be one formed from zig-zag or undulating wire or wires. Depicting a non-limiting example, Figure 3 discloses an exploded or enlarged view of a braided stent **300** with braiding of the stent filaments **320**. As used herein, the term braiding and its variants refer to the diagonal intersection of elongate filaments **320** so that each filament passes alternately over and under one or more of the other filaments, which is commonly referred to as an intersection repeat pattern. Useful braiding patterns include, but are not limited to, a diamond braid having a 1/1 intersection repeat pattern, a regular braid having a 2/2 intersection repeat pattern or a Hercules braid having a 3/3 intersection repeat pattern. The passing of the filaments under and over one and the other results in slidable filament crossings that are not inter-looped or otherwise mechanically engaged or constrained. The present invention is not limited by the above embodiment. For example, some or all of the filaments may be twisted at the filament or wire crossings. Ends of stent **300** may be atraumatic.

While the stent **300** may be formed of metals, plastics or any other materials, it is preferred that a biocompatible material or construction is employed. Useful biocompatible materials include, but are not limited to, biocompatible metals, biocompatible alloys, biocompatible polymeric materials, including synthetic biocompatible polymeric materials and bioabsorbable or biodegradable polymeric materials, materials made from or derived from natural sources and combinations thereof. Useful biocompatible metals or alloys include, but are not limited to, Nitinol, stainless steel, cobalt-based alloy such as Elgiloy, platinum, gold, titanium, tantalum, niobium, polymeric materials and combinations thereof. Useful synthetic biocompatible polymeric materials include, but are not limited to, polyesters, including polyethylene terephthalate (PET) polyester filaments, polypropylenes, polyethylenes, polyurethanes, polyolefins, polyvinyls, polymethylacetates, polyamides, naphthalane dicarboxylene derivatives, silks and polytetrafluoroethylenes. The polymeric materials may further include a metallic, a glass, ceramic or carbon constituent or fiber. Useful and nonlimiting examples of bioabsorbable or biodegradable polymeric materials include poly(L-lactide) (PLLA), poly(D,L-lactide) (PLA), poly(glycolide) (PGA), poly(L-lactide-co-D,L-lactide) (PLLA/PLA), poly(L-lactide-co-glycolide) (PLLA/PGA), poly(D,L-lactide-co-glycolide) (PLA/PGA), poly(glycolide-co-trimethylene carbonate) (PGA/PTMC), polydioxanone (PDS), Polycaprolactone (PCL), polyhydroxybutyrate (PHBT), poly(phosphazene) poly(D,L-lactide-co-caprolactone) PLA/PCL), poly(glycolide-co-caprolactone) (PGA/PCL), poly(phosphate ester) and the like. Further, the stent 300 may include materials made from or derived from natural sources, such as, but not limited to collagen, elastin, glycosaminoglycan, fibronectin and laminin, keratin, alginate, combinations thereof and the like. Further, in some applications, the stent **300** may desirably be embedded in a coating of silicone. Additional details of such desirable stents are described in U.S. Pat. No. 6,162,244.

Further, the stent **300** may be made from polymeric materials which may also include radiopaque materials/markers to improve external imaging under magnetic resonance imaging (MRI) and/or ultrasonic visualization techniques. In essence, the stent **300** may be selectively made radiopaque at desired areas along the stent or made be fully radiopaque, depending on the desired end-product and application. Such radiopaque materials include complex metallic alloys, metallic-based powders or ceramic-based powders, particulates or pastes which may be incorporated into the polymeric material. For example, the radiopaque material may be blended with the polymer composition from which the polymeric wire is formed, and subsequently fashioned into the stent as described herein. Specifically, materials for enhancing MRI visibility include, but are not limited to, metal particles of gadolinium, iron, cobalt, nickel, dysprosium, dysprosium oxide, tantalum, gold, iridium, platinum, palladium, cobalt based alloys, iron based alloys, stainless steels, or other paramagnetic or ferromagnetic metals, gadolinium salts, gadolinium complexes, gadopentetate dimeglumine, compounds of copper, nickel, manganese, chromium, dysprosium and gadolinium. The stent 300 may have an inner core of tantalum, gold, platinum, iridium or combination of thereof and an outer member or layer of Nitinol to provide a composite filament for improved radiopacity or visibility. Alternatively, the radiopaque material may be applied to the surface of the metal or polymer stent. Various radiopaque materials and their salts and derivatives may be used including, without limitation, bismuth, barium and its salts such as barium sulfate, tantalum, tungsten, gold, platinum and titanium, to name a few. Additional useful radiopaque materials and other features may be found in U.S. Pat. No. 6,626,936.

Also, the stent **300** may have coverings, films, coatings, and the like disposed over, under or throughout or embedding the stent **300.** For example, as depicted in Figure 4, the stent **300** may include a covering **340.** In some embodiments a polymeric covering, disposed over the longitudinal length or a portion of the longitudinal length of the stent **300.** Further, as depicted in Figure 5, the stent **300** may include a liner **360,** desirably a polymeric liner, disposed within the longitudinal length or a portion of the longitudinal length of the stent **300.** Moreover, as depicted in Figure 6, the stent **300** may include a both a covering **340** and a liner **360,** desirably a polymeric covering and liner which include the same or different polymeric materials, disposed over and within the longitudinal length or a portion of the longitudinal length of the stent **300.** Other features, which may be included with the stent **300** of the present invention, include surface modification for ultrasound, cell growth or therapeutic agent delivery; varying stiffness of the stent or stent components; varying geometry, such as tapering, flaring, bifurcation and the like; varying material; varying geometry of stent components, for example tapered stent filaments; and the like.

The covering and the liner of Figure 6 may be a unitary film or coating that embeds or partially embeds the stent **300.** The coating or coatings may be on the stent **300,** components of the stent **300,** and combinations thereof. The covering **340** and/or the liner **360** may be in the form of a tubular structure, for example composed of polymeric material and/or silicone. The covering **340** and/or the liner **360** may also comprise any plastic or polymeric material, desirably a somewhat hard but flexible plastic or polymeric material. The covering **340** and/or the liner **360** may be transparent or translucent, desirably substantially or partially transparent. The stent components, in part or in total, may be temporary, for example bioabsorbable, biodegradable, and the like, or may be permanent (i.e., not substantially bioabsorbable or biodegradable), for example the above-described biocompatible metals, alloys and polymers.

Furthermore, the covering **340** and/or the liner **360** may be constructed of any suitable biocompatible materials, such as, but not limited to, polymers and polymeric materials, including fillers such as metals, carbon fibers, glass fibers or ceramics. Useful covering **340** and/or the liner **360** materials include, but are not limited, polyethylene, polypropylene, polyvinyl chloride, polytetrafluoroethylene (PTFE), including expanded polytetrafluoroethylene (ePTFE), fluorinated ethylene propylene, fluorinated ethylene propylene, polyvinyl acetate, polystyrene, poly(ethylene terephthalate), naphthalene dicarboxylate derivatives, such as polyethylene naphthalate, polybutylene naphthalate, polytrimethylene naphthalate and trimethylenediol naphthalate, polyurethane, polyurea, silicone rubbers, polyamides, polyimides, polycarbonates, polyaldehydes, polyether ether ketone, natural rubbers, polyester copolymers, styrene-butadiene copolymers, polyethers, such as fully or partially halogenated polyethers, silicones, and copolymers and combinations thereof.

Further, the stent **300** may be treated with a therapeutic agent or agents, such as, but not limited to, anti-thrombogenic agents (such as heparin, heparin derivatives, urokinase, and PPack (dextrophenylalanine proline arginine chloromethylketone); anti-proliferative agents (such as enoxaprin, angiopeptin, or monoclonal antibodies capable of blocking smooth muscle cell proliferation, hirudin, and acetylsalicylic acid); anti-inflammatory agents (such as dexamethasone, prednisolone, corticosterone, budesonide, estrogen, sulfasalazine, and mesalamine); antineoplastic/antiproliferative/anti-miotic agents (such as paclitaxel, 5-fluorouracil, cisplatin, vinblastine, vincristine, epothilones, endostatin, angiostatin and thymidine kinase inhibitors); anesthetic agents (such as lidocaine, bupivacaine, and ropivacaine); anti-coagulants (such as D-Phe-Pro-Arg chloromethyl keton, an RGD peptide-containing compound, heparin, antithrombin compounds, platelet receptor antagonists, antithrombin antibodies, anti-platelet receptor antibodies, aspirin, prostaglandin inhibitors, platelet inhibitors and tick antiplatelet peptides); vascular cell growth promotors (such as growth factor inhibitors, growth factor receptor antagonists, transcriptional activators, and translational promotors); vascular cell growth inhibitors (such as growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin); cholesterol-lowering agents; vasodilating agents; and agents which interfere with endogenous vascoactive mechanisms.

Further, as depicted in Figure 7, the stent **300** according to the current invention may have a straight or substantially straight longitudinal portion **380.** The present invention, however, is not so limited. For example, the stent **300** may have a varied diameter, such as a flaring or tapering, along a portion or portion of its longitudinal expanse. Further, the stent **300** may include anti-migration features such as flares, steps, ridges, anchors, fasteners or other protrusions on its external surface. One non-limiting example of a varied diameter stent **300** is depicted in Figure 8. The stent **300** of Figure 8 may include a longitudinal length **380** and one or two flared ends **400.** As depicted in Figure 8, the flared ends **400** may include enlarged flared ends having a diameter greater than the diameter of the longitudinal portion **380** of the stent **300.** However, alternatively, the flared ends **400,** individually or in combination, may have a smaller diameter than the diameter of the longitudinal portion **380** of the stent **300.**

Additionally, the stent **300** itself may include anti-migration features such as flares, steps, ridges, anchors, fasteners, etc. Further, the stent **300** may be repositionable, removable and/or reconstrainable, and/or may include multiple interconnected or non-interconnected filaments. For example, the stent **300** may include a filament loop or element, such as a suture loop or element, a polymeric loop or element, metallic or element, and combinations thereof. Such additional loop or element may be accessible to a practitioner, for example by the use of forceps, to reposition, remove and/or reconstrain the stent **300** after it has been delivered, partially or totally, to a bodily lumen. Moreover, a loop or element may be integrally formed as part of the stent **300.** Further details of useful repositioning, removing and/or reconstraining loops or elements may be found in U.S. Patent Application No. 11/341,540, filed January 27, 2006 and entitled "Stent Retrieval Member And Devices And Methods For Retrieving Or Repositioning A Stent" (U.S. Pub. No. 2006/0190075 A1) and in U.S. Patent Application No. 11/432,065, filed May 11, 2006, attorney docket 792-32, and entitled "Integrated Stent Respostioning And Retrieval Loop" (U.S. Pub. No. 2006/0276887 A1).

The method of utilizing the system **100** is also contemplated by the present invention. The utilization includes a method for loading a stent **300** utilizing the system **100** in percutaneous, transluminal or other insertion techniques. The system **100** allows the practitioner to easily load a stent into a delivery system **100** with minimal effort and without damaging the stent **300.**

In an initial setting, the stent delivery system **100** can be supplied to the practitioner in a protective package. The stent **300** in the package may be supplied in an unconstrained condition (so the plastic stent does not take a "set"). When ready to be used, the practitioner may take the delivery system **100** and the stent **300** out of the package in preparation of the procedure. As discussed above, a packaged delivery deployment system **100** typically may include an external member **160,** an intermediate member **140,** and an inner member **120,** each having opposed distal and proximal ends with respective handles **170, 150,** and **130.** Each of these handles **170, 150,** and **130** control the respective member's slidable disposition; optionally, a stent basket **200** may be securely disposed to the distal end of the stent loading intermediate member **140.**

The handles **130, 150,** and **170** may be supplied constrained in the package. Such constraint may be provided, for instance, by the way of friction or mechanical locks, to prevent the practitioner from accidentally moving the handles **130,150** and **170.** Out of the package, the shaft portion **155** of the intermediate handle **150** may be provided partly in the receptacle **134** of the proximal handle **130** through a mechanical and locking mechanism. In this position, the distal end **139** of the intermediate member **140** is extended away from the practitioner to an extended (distal) position **153.** In such distal position **153,** the stent basket **200** attached to the distal end **139** of the intermediate member **140** may be in an unconstrained position and readily accept a stent **300.**

The practitioner may place the stent **300** over the stent holder **210** provided at distal end **139** of the intermediate member **140** as follows. First, the proximal end of the stent **300** may be placed within the stent basket **200,** as depicted in Figure 15. Afterwards, the stent **300** may be squeezed or radially contacted onto or about the inner member **120.** The stent **300** may then be pushed into the stent basket **200** to facilitate its engagement within the intermediate member **140.** Alternatively, the stent **300** may be disposed within a loading cartridge (not shown) for facilitating storage and delivery of the stent **300** into the intermediate member **140.** The loading cartridge may contain a piston or other axially movable member to facilitate stent movement. Details of suitable stent loading cartridges are further described in U.S. Pat. No. 6,068,635 and/or U.S. Patent Application Publication 2003/0083730 A1.

During the loading of the stent **300,** the handles **170** and **150** may be kept fixed in relatively constant axial displacement from one and another. As such, the inner member **120** and the intermediate member **140** may also be kept in relative constant axial positions with the intermediate member **140** being substantially disposed within the external member **160.** However, the intermediate member **140** need not be completely contained within the external member **160.** Rather, a portion of the distal end of the intermediate member **140** may be axially outside or distally disposed from the distal end of the external member **160.** Additionally, the smaller distal opening or flange of the intermediate handle **150** may serve as a stop or an axially limiting mechanism. Such limiting mechanism keeps the inner and intermediate members **120,** 1**40** in relative constant axial arrangement during loading of the stent **300.** To complete the stent **300** loading, the proximal handle **130** may be pulled away from the distal handle **170** to complete the loading.

The intermediate basket loading member is fully utilized. In the embodiment of this invention illustrated by Figures 9 and 10, the practitioner may load the stent **300** by sliding the distal handle **170** in a distal direction. This action can slide the external member **160** onto the stent **300** to load and position the stent **300** well into the distal end of the external member **160.** The practitioner then may move the distal handle **170** further distally to constrain the stent **300** until the stent can be fully constrained by the external member **160.** The external member **160,** attached to the distal handle **170,** can then slide over the stent basket **200** and collapse and constrain the stent 300 completely over the inner member **120.**

The present invention may also include a method of providing the tubular band **220** disposed toward the distal end of the inner member **120.** Such method may releasably secure the stent **300** in the stent deployment region **110** between the inner and members **120, 160.** Moreover, the method, in an example not part of the invention, may further include axially sliding the external member **160** toward a proximal position for releasing the stent **300** from the stent deployment region **110.** The method may yet further include providing a distal handle **170** disposed at the proximal end **157** of the external member **160;** providing a proximal handle **130** disposed at the proximale end **117** of the inner member **120;** and providing a stent loading intermediate handle **150** disposed at the proximal end **137** of the intermediate member **140.** The method of independently moving the external member **160,** the inner member **120** or the intermediate member **140** may be achieved by manual manipulation of the handles **170, 130, 150.**

The practitioner may manipulate the handles **130, 150,** and **170** as follows: As depicted in Figure 10, the practitioner may grasp the proximal handle **130** and the intermediate handle **150** with two hands. The practitioner may, then, utilize the intermediate handle lock and may actuate a release mechanism to release the intermediate handle **150** from the protrusion **136.** This act retracts the intermediate handle **150** into the proximal handle **130.** This retracts the intermediate member **140** relative to core inner member **120.** The system **100** may be configured such that mechanism **154** can be overcome when retracting the basket **200.** As a result, the stent **300** can be positioned within the external member **160** with the portions overlying the basket **200.**

Thus formed, the practitioner may slide the distal handle **170** in the direction away from the intermediate handle **150** and the proximal handle **130.** This can actuate the external member **160** to extend over the stent basket **200** along with the stent **300** lying therein which collapses into a constrained position inside the external member **160.** Once the external member **160** completely engages the stent basket **200,** the practitioner can pull back the intermediate handle **150** in relation to the proximal handle **130** and the distal handle **170.** As the intermediate handle **150** is pulled back, the inner member **120** keeps the stent from traveling back as well. Thus, the stent basket **200** releases the stent **300** within the external member, which then retain the stent there within. Thus, the stent may be properly loaded into the system **100.** Once the intermediate handle **150** is pulled back completely, the mechanical lock **136** and the protrusion **152** combination locks the position of the intermediate handle **150** in step with the proximal handle **130** so that the intermediate handle and the proximal handle now form a single handle. The delivery system can now be ready to be inserted into the body.

In some embodiments, the delivery system as depicted in Figure 10, the stent basket **200** may be moved away from the constrained stent **300.** Afterwards, the stent **300** may be completely loaded or contained within the external member **160,** and the intermediate handle **150** may be advanced proximally away from the distal handle **170** and toward the proximal handle **130** until it locks into the proximal handle **130.** This effectively moves the stent basket **200** axially away from the loaded stent **300.** In other words, the proximal end of the loaded stent **300** can be now free from the stent basket **200.** Such removal of the stent basket **200** from the loaded stent **300** facilitates the delivery of the stent **300** as less force may be required to deploy the stent **300.** Also, this ensures that the stent basket **200** will not interfere with the stent **300** while the stent is being deployed. Now, only the distal handle **170** needs to be manipulated to deploy the stent **300.** In some embodiments, the handles may be designed with a less accessible intermediate handle release mechanism (basket retraction handle). Alternatively, the practitioner can control the position of the intermediate handle by another feature such as a rod, which may interlock with the proximal handle **130.** Also, the retention of the intermediate handle **150** can be made permanent by features such that the handles cannot be separated if this feature is desirable.

As depicted in Figure 9 and as described above, the stent **300** can be fully loaded into the system **100** of the present invention. Thus positioned, the stent holder **210** releasably secures the stent **300** between the inner member **120** and the external member **160.** In some embodiments, the stent holder **210** may be a hollow tubular band. More desirably, the stent holder **210** may be a hollow tubular band that may be free or substantially free of barbs, pins or protrusions which may engage and possible damage the stent **300.** The stent holder **210** may be made of any suitable polymeric, rubber or metallic material. In some embodiments, the stent holder **210** may be a raised portion of the inner member **120.** Moreover, the stent holder **210** may have adhesives or a pattern, such as a surface pattern of indentations and/or protrusions, for facilitating securement of the stent **300.** In some embodiments, the stent holder **210** may have barbs, pins or protrusions which may engage the stent **300.** Further, with any of the embodiments, the system **100** may include multiple stent holders **210,** either axially spaced apart or axially juxtaposed. Further, the stent holder **210** may not have to completely encompass the inner member **120,** but may be only partially disposed around a circumferential portion of the inner member **120**.

The stent delivery system **100** can now be positioned in the patient and the stent **300** deployed by moving the distal handle **170** in a proximal direction. The intermediate handle **150** may have a release mechanism such that the intermediate handle **150** can be repositioned back to the original position if the stent **300** needs to be re-loaded. If needed, the stent **300** can be removed from the body and reloaded on the delivery system **100** by unlocking the intermediate handle and returning it to the distal position. Optionally, the system can be positioned by axially moving or sliding the stent engaging basket **200** to a location past the stent deployment region **110** for disengagement of the stent **300** from the intermediate member **140.**

The practitioner can next perform the insertion of the distal end of the system **100** into a patient's body with a lead-in such as the distal end tip **240.** Once the practitioner navigates the distal end tip **240** to a desired location, and is satisfied with the location and orientation of the partially deployed stent **300,** the practitioner can actuate the handle **170** to fully deploy the stent **300** as depicted in Figure 33. The practitioner can then pull back the distal handle **170** toward the intermediate handle **150,** thereby pulling back the external member **160.** This step uncovers the constrained stent **300** and may be designed to unload the stent into the desired deployment region. The delivery system **100** can then be removed from the body.

As depicted in Figure 11, the loaded stent **300** may be unloaded to a bodily lumen (not shown) by advancing the distal handle **170** and correspondingly the external member **160** axially away from the distal tip **240.** When the practitioner partially retracts the tubular member **160** to a position as shown in Figure 25, the distal portion of the stent **300** beyond the sheath may expand. The practitioner can use fluoroscopy, MRI, endoscopy or other minimal invasive viewing techniques and apparatus to determine whether the stent **300** is appropriately positioned. Once the system **100** is properly inserted, the practitioner may position the distal end tip **240** at a selected location in a patient's vessel for deployment of the stent **300.** This can be accomplished by a various means, in some embodiments by providing markers such as radiopaque rings or indicia on the core part proximate the distal end tip **240** to properly locate the system **100.**

Some factors, which are only apparent upon deploying the stent, can render the location initially thought to be inappropriate, end up as the most optimal location. For these reasons, the embodiment of Figure 1 may include indicia there on, so that a practitioner using any number of imaging methods such as fluoroscopy, MRI, ultrasound, etc. may ascertain the extent of deployment of the stent **300,** as more fully discussed below. The practitioner may ascertain whether the stent **300** is properly positioned by any number of imaging methods before fully deploying the stent **300** and retract the partially deployed stent **300.** This can be important to both the practitioner and the patient as improper positioning can increase the trauma and risk to a patient and curtail the efficiency of the treatment. If the stent **300** is not properly positioned, the practitioner may displace the handle portion **130** proximally to retract the core inner member **120** and the intermediate member **140** fully within the tubular member **160.** Such displacement can ensure that the stent **300** returns to the condition as depicted in Figure 10. The distal end can then be maneuvered to another desired location or into a desired orientation and the process repeated.

As depicted in Figures 10 and 11 and described above, the intermediate handle **150** and the proximal handle **130** may be proximally and/or juxtaposingly disposed during certain stages of loading, constraining and/or deploying the stent **300.** Accordingly, the stent loading intermediate handle **150** may be integrated, for example mechanically integrated, with the proximal handle **130** to permit concurrent or simultaneous movement of the two handles **130, 150.** Such mechanical integration, may be achieved by matching and/or interlocking mechanisms (Figure 1) on the two handles **150, 130.** The mechanical integration may be achieved through various means such as interference fit, adhesive fit, thread fit, soft material that compresses to fit the intermediate handle **150** into the proximal handle **130,** any other equivalent fit thereof. In some embodiments, the use of releasably interlocking mechanisms. (Figure 1) on the two handles **130, 150** may provide the mechanism integration. This mechanism may permit independent movement of the two handles **130, 150** by mechanically releasing the mechanisms from one and another. It may also permit concurrent or simultaneous movement of the two handles **130, 150** by mechanically engaging the mechanisms with one and another. In some embodiments, the fit between the handles may be interference fit or could have a soft material that compresses to fit intermediate handle into proximate handle. Further, other embodiments may be adhesive, or threads, etc.

Figures 21 to 24 depict another embodiment of this invention. Figure 22 shows an enlarged view of an embodiment of the proximal portion of the system **100** of the present invention. The handle interlock and the protrusion **52** may be a single-molded in the design of the intermediate handle. A step or cut-away portion of the inner member **120** may optionally serve as the above-described proximal portion. As described, such a proximal portion in conjunction with the small proximal opening **440** of the intermediate handle **150** serves as a stop during loading of the stent **300** into the system **100** of the present invention.

Another feature of the present invention is that the stent loading can be reversible. If the practitioner suspects that stent **300** was incorrectly positioned during loading, or determines that a different stent should be used, stent **300** can be easily unloaded, by operating handles **130** and **150** to advance inner member **120** toward the open position. This progressively releases stent **300** from the external member **160,** whereupon the stent **300** may be removed from stent basket **200** by hand. Alternatively, if the practitioner needs to adjust the position of deployment or retract the stent, the practitioner may push the distal handle **170** away from the intermediate handle **150** to move the external member over the stent. Then, the practitioner is free to either move the stent to another deployment region or take it out completely. Further, the external member **160** may be retracted proximally past the tubular band **220,** thereby allowing repositioning of the stent **300** within the external member **160** and/or over the inner member **120.** The external member **160** may be re-advanced over the stent **300** and the tubular band **220** to releasably and securely reset the position of the stent basket **200** and/or the intermediate member **140** relative to the position of the inner member **120,** thereby allowing reconstrainment of the stent.

The above described assembly may be suited for medical applications such as in the gastrointestinal tract, the biliary tract, the urinary tract, and the respiratory tract, as long as said medical applications can be suitable for the method disclosed in claim 15. The assembly in accordance with the present invention, however, could also be used in the neurological system (e.g., in the brain), the vascular system (e.g., in arteries or veins), in the cardiovascular system (e.g., in the heart) and in the like. Further, the assembly may be used in artificially created passageways as well. Reference to bodily passageways may be to passageways in any of the aforementioned tracts and systems or elsewhere in the body. In some embodiments, the assembly may be used in artificially created passageways.

The stent delivery system **100** is typically supplied to the practitioner in a protective package (not shown). The stent **300** may be supplied in an unconstrained condition within the package or may be supplied separately from the package. As depicted in Figure 30, intermediate handle **150** is in a distally extended position with respect to the proximal handle **130.** With the intermediate handle **150** so distally extended, the stent basket **200** is also is a relative distal position such that it may accept the stent **300**. As previously described, the handles **130, 150, 170** are designed so that in the as-packaged condition, their relative movement is restricted or minimized either by friction or mechanical locks.

As depicted in Figure 31, after the stent **300** is loaded into the delivery system **100,** the distal handle **170** is moved distally to constrain the stent **300** until the stent **300** is fully constrained by the outer catheter or outer exterior member **160.**

As depicted in Figure 32, the stent basket **200** is then retracted by sliding the intermediate handle **170** in a proximal direction towards the proximal handle **130** until the intermediate handle **150** locks into the proximal handle **130** and the basket **200** is no longer in contact with the stent **300.** After the intermediate handle **150** is locked into the proximal handle **130,** advantageously there is now only one handle which may be manipulated to deploy the stent **300.**

The stent delivery device **100** may then be positioned in a patient by a practitioner. As depicted in Figure 33, the stent **300** may be deployed by a practitioner by moving the proximal; handle **130** in a proximal direction toward handles **130, 150.** If needed, the stent **300** may be removed from the body or repositioned within the body by reloading the stent **300** onto the delivery device **100.** Such reloading may be accomplished by unlocking the intermediate handle **150,** for instance by actuating mechanism **154a,** and moving the intermediate handle **150** distally away from the proximal handle **130.**

As depicted in Figure 34, the handle interlock **152** prevents or inhibits unwanted movement of the intermediate handle **150** from moving too far distally away from the proximal handle **130.** Further, the protrusion **136** and ledge **156a** aids in the positioning of the stent basket **200** for proper stent placement during constraining of the stent **300** within the delivery system **100.** As depicted in Figure 25, the intermediate handle **150** is slidable into the proximal handle and may be releasably contained therein.

As depicted in Figure 35, the handle interlock **152'** may be, if desired, a single molded feature of the intermediate handle **150.**

As depicted in Figure 36, the positioning of the intermediate handle **150,** which serves in part as a basket **200** retraction handle, my be controlled by another feature, such as a rod **180** which interlocks with the proximal handle **130.** Such a rod **180** may also provide limited motion of the intermediate handle, as depicted in Figure 26.

The following embodiments or aspects of the invention may be combined in any fashion and combination and be within the scope of the present invention, as follows:
Embodiment 1. A delivery catheter handle assembly comprising: a proximal handle attached to a proximal end of an axially elongated inner member; an intermediate handle attached to a proximal end of in axially elongated intermediate member, at least one of said proximal and said intermediate handles being designed to accommodate and engage the other of said proximal and said intermediate handles; and a distal handle attached to a proximal end of an axially elongated external member overlying at least a portion of said intermediate member.
Embodiment 2. The delivery catheter handle assembly according to embodiment 1, wherein said proximal handle and said intermediate handle include a handle-interlock mechanism, a portion of said intermediate handle being releasably retained by said proximal handle to prevent distal movement of said intermediate handle.
Embodiment 3. The delivery catheter handle assembly according to embodiment 2, wherein said handle-interlock mechanism is molded unitary with said proximal handle and said intermediate handle.
Embodiment 4. The delivery catheter handle assembly according to embodiment 1, further comprising: a slide-and-lock mechanism, wherein one of said proximal handle and said intermediate handle is retracted into the other to define a unitary retracted position configured to limit travel between said proximate handle and said intermediate handle.
Embodiment 5. The delivery catheter handle assembly according to embodiment 4, wherein said intermediate handle includes a release mechanism such that the intermediate handle can be released and positioned relatively away from said proximal handle to permit re-loading of a prosthesis.
Embodiment 6. The delivery catheter handle assembly according to embodiment 4, wherein said slide-and-lock mechanism is molded unitarily with said proximal handle and said intermediate handle.
Embodiment 7. The delivery catheter handle assembly according to embodiment 1, wherein said intermediate handle includes a mechanism at a distal position configured to prevent unintended retraction.
Embodiment 8. The delivery catheter handle assembly according to embodiment 1, wherein said intermediate handle includes at least one projecting finger for interlockingly connecting with said proximal handle.
Embodiment 9. The delivery catheter handle assembly according to embodiment 1, wherein said intermediate handle includes a gripping surface.
Embodiment 10. The delivery catheter handle assembly of embodiment 9, wherein said gripping surface includes wings.
Embodiment 11. The delivery catheter handle assembly ofembodiment 9, wherein said gripping surface includes a soft grip.
Embodiment 12. The delivery catheter handle assembly of embodiment 1, further including a guidewire port.
Embodiment 13. The delivery catheter handle assembly according to embodiment 1, wherein said proximal handle includes projections to provide frictional force retaining said intermediate handle during the prosthesis loading process.
Embodiment 14. The delivery catheter handle assembly according to embodiment 1, wherein said proximal handle includes a mechanism for interlockingly capturing said intermediate handle during the prosthesis loading process.
Embodiment 15. The delivery catheter handle assembly according to embodiment 1, wherein in an as-packaged condition, the relative movement of the handles is mechanically limited.
Embodiment 16. The delivery catheter of embodiment 1, wherein said intermediate handle further includes a locking mechanism for connecting with said proximal handle, said locking mechanism having a release mechanism which is recessed.
Embodiment 17. An apparatus for loading and delivering a prosthesis, the apparatus comprising: a prosthesis; a core segment including a proximal handle, an axially extended inner member formed integral with said proximal handle; a middle segment including an intermediate handle, an axially elongated intermediate member formed integral with said intermediate handle, wherein one of said proximal and said intermediate handles are designed to assume and engage other of said proximal and said intermediate handles; and an external segment including a distal handle, an axially elongated external member formed integral with said distal handle and overlying at least a portion of said intermediate member.
Embodiment 18. The apparatus according to embodiment 17, wherein said proximal handle and said intermediate handle include a handle-interlock mechanism, wherein a portion of said intermediate handle is releasably retained by said proximal handle.
Embodiment 19. A method for loading prosthesis onto a delivery system, the method comprising: providing a prosthesis delivery system including a proximal handle attached to an inner member, an intermediate handle attached to an intermediate handle, one of said proximal and said intermediate handles being designed to accommodate and engage another of said proximal and said intermediate handles, and a distal handle attached to an external member overlying at least a portion of said intermediate member, said proximal handle having a releasable locking mechanism for releasably engaging said intermediate handle; providing a prosthesis in a unconstrained condition; positioning said intermediate handle in a position away from said proximal handle and loading said prosthesis; moving said distal handle distally to constrain said prosthesis within said external member; and retracting said prosthesis basket into said axially elongated intermediate member by sliding said intermediate handle in a proximal direction until it locks into said proximal handle via said releasable locking mechanism and said basket is no longer in contact with said prosthesis.
Embodiment 20. The method for loading prosthesis according to embodiment 19, the method (not part of the invention) further including the steps of: positioning said prosthesis delivery system into a patient; and deploying said prosthesis by moving said distal handle in a proximal direction.
Embodiment 21. The method for loading prosthesis according to embodiment 20, the method (not part of the invention) further including the steps of: unlocking said locking mechanism and returning said intermediate handle to said distal position to reload said deployed prosthesis on said delivery system and remove or reposition said prosthesis.

While select preferred embodiments of this invention have been illustrated and/or described herein, those skilled in the art will appreciate that many modifications and variations of the present invention may occur and therefore it is to be understood that these modifications are incorporated within these embodiments as if they were fully illustrated and described herein without departing from the scope of the appended claims.

## Claims

1. A delivery catheter handle assembly (100) comprising:
a proximal handle (130) attached to a proximal end (117) of an axially elongated inner member (120);
an intermediate handle (150) attached to a proximal end (137) of an axially elongated intermediate member (140), 150) being designed to accommodate and engage the other of said proximal and said intermediate handles (130, 150); and
a distal handle (170) attached to a proximal end (157) of an axially elongated external member (160) overlying at least a portion of said intermediate member (140),
the assembly further comprises a basket (200) with opposed proximal and distal ends, wherein said proximal end of the basket can be securely disposed to the distal end of the intermediate handle (150)
**characterized in that**
at least one of said proximal and said intermediate handles (130, 150) being designed to accommodate and engage the other of said proximal and said intermediate handles (130, 150)
said proximal handle (130) comprises an opening (132) at its distal end (129) defining a receptacle (134) extending significantly into said proximal handle (130), and
said intermediate handle (150) comprises a proximal shaft portion (155, 155') and a distal flange portion (157a),
where said proximal shaft portion (155, 155') of said intermediate handle (150) is slidable into and out of said receptacle (134) of said proximal handle (130) and
where said distal flange portion (157a) serves as a stop for insertion of said intermediate handle (150) into said receptacle (134) of said proximal handle (130) further comprises a basket (200) with opposed proximal and distal ends, wherein said proximal end of the basket can be securely disposed to the distal end of the intermediate handle (150).

2. The delivery catheter handle assembly (100) according to claim 1, wherein said proximal handle (130) and said intermediate handle (150) include a handle-interlock mechanism (152, 152'), a portion of said intermediate handle (150) being releasably retained by said proximal handle (130) to prevent distal movement of said intermediate handle (150).

3. The delivery catheter handle assembly (100) according to claim 2, wherein said handle-interlock mechanism (152, 152') is molded unitary with said proximal handle (130) and said intermediate handle (150).

4. The delivery catheter handle assembly (100) according to claim 1, further comprising:
a slide-and-lock mechanism (136, 154, 156, 152, 152'), wherein said intermediate handle (150) is retracted into the proximal handle to define a unitary retracted position configured to limit travel between said proximal handle (130) and said handle intermediate handle (150).

5. The delivery catheter handle assembly (100) according to claim 4, wherein said intermediate handle (150) includes a release mechanism (158) such that the intermediate handle (150) can be released and positioned relatively away from said proximal handle (130) to permit re-loading of a prosthesis (300).

6. The delivery catheter handle assembly (100) according to claim 4, wherein said slide-and-lock mechanism (136, 154, 156, 152, 152') is molded unitarily with said proximal handle (130) and said intermediate handle (150).

7. The delivery catheter handle assembly (100) according to claim 1, wherein said intermediate handle (150) includes a mechanism (154, 154a) at a distal position (155') configured to prevent unintended retraction.

8. The delivery catheter handle assembly (100) according to claim 1, wherein said intermediate handle (150) includes at least one projecting finger for interlockingly connecting with said proximal handle (130).

9. The delivery catheter handle assembly (100) according to claim 1, wherein said intermediate handle (150) includes a gripping surface, preferably wings (169) or a soft grip.

10. The delivery catheter handle assembly (100) of claim 1, further including a guidewire port.

11. The delivery catheter handle assembly (100) according to claim 1, wherein said proximal handle (130) includes projections (127) to provide frictional force retaining said intermediate handle (150) during the prosthesis loading process.

12. The delivery catheter handle assembly (100) according to claim 1, wherein said proximal handle (130) includes a mechanism for interlockingly capturing said intermediate handle (150) during the prosthesis loading process.

13. The delivery catheter handle assembly (100) according to claim 1, wherein in an as-packaged condition, the relative movement of the handles (130, 150, 170) is mechanically limited.

14. The delivery catheter handle assembly (100) of claim 1, wherein said intermediate handle (150) further includes a locking mechanism for connecting with said proximal handle (130), said locking mechanism having a release mechanism which is recessed.

15. A method for loading prosthesis (300) onto a delivery system (100), the method comprising:
providing a prosthesis delivery system (100) including a proximal handle (130) attached to an inner member (120), an intermediate handle (150) attached to an intermediate member (140), one of said proximal and said intermediate handles (130, 150) being designed to accommodate and engage another of said proximal and said intermediate handles (130, 150), and a distal handle (170) attached to an external member (160) overlying at least a portion of said intermediate member (140), said proximal handle (130) having a releasable locking mechanism for releasably engaging said intermediate handle (150);
providing a prosthesis (300) in a unconstrained condition;
positioning said intermediate handle (150) in a position away from said proximal handle (130) and loading said prosthesis (300) in a prosthesis basket (200) wherein said basket (200) having opposed proximal and distal ends and said proximal end of the basket can be securely disposed to the distal end of the intermediate handle (150);
moving said distal handle (170) distally to constrain said prosthesis (300) within said external member (160); and
retracting said prosthesis basket (200) into said axially elongated intermediate member (140) by sliding said intermediate handle (150) in a proximal direction until it locks into said proximal handle (130) via said releasable locking mechanism and said basket (200) is no longer in contact with said prosthesis (300),
**characterized in that**
said proximal handle (130) comprises an opening (132) at its distal end (129) defining a receptacle (134) extending significantly into said proximal handle (130), and
said intermediate handle (150) comprises a proximal shaft portion (155, 155') and a distal flange portion (157a),
where said proximal shaft portion (155, 155') of said intermediate handle (150) is slidable into and out of said receptacle (134) of said proximal handle (130) and
where said distal flange portion (157a) serves as a stop for insertion of said intermediate handle (150) into said receptacle (134) of said proximal handle (130).

## Patentansprüche

1. Abgabekatheter-Griff-Anordnung (100), die aufweist:
einen proximalen Griff (130), der an einem proximalen Ende (117) eines axial länglichen Innenteils (120) angebracht ist;
einen Zwischengriff (150), der an einem proximalen Ende (137) eines axial länglichen Zwischenteils (140) angebracht ist; und
einen distalen Griff (170), der an einem proximalen Ende (157) eines axial länglichen Außenteils (160) angebracht ist, das über mindestens einem Abschnitt des Zwischenteils (140) liegt,
wobei die Anordnung ferner einen Korb (200) mit einem entgegengesetzten proximalen und distalen Ende aufweist,
wobei das proximale Ende des Korbs am distalen Ende des Zwischengriffs (150) fest angeordnet werden kann,
**dadurch gekennzeichnet, dass**
mindestens eine Komponente aus dem proximalen und dem Zwischengriff (130, 150) so gestaltet ist, dass sie die andere Komponente aus dem proximalen und dem Zwischengriff (130, 150) unterbringt und ergreift,
der proximale Griff (130) eine Öffnung (132) an seinem distalen Ende (129) aufweist, die eine Aufnahme (134) bildet, die sich in den proximalen Griff (130) erheblich erstreckt, und
der Zwischengriff (150) einen proximalen Schaftabschnitt (155, 155') und einen distalen Flanschabschnitt (157a) aufweist,
wobei der proximale Schaftabschnitt (155, 155') des Zwischengriffs (150) in die Aufnahme (134) des proximalen Griffs (130) und aus ihr verschiebbar ist und
wobei der distale Flanschabschnitt (157a) als Anschlag zur Einführung des Zwischengriffs (150) in die Aufnahme (134) des proximalen Griffs (130) dient.

2. Abgabekatheter-Griff-Anordnung (100) nach Anspruch 1, wobei der proximale Griff (130) und der Zwischengriff (150) einen Griffverriegelungsmechanismus (152, 152') aufweisen, wobei ein Abschnitt des Zwischengriffs (150) durch den proximalen Griff (130) lösbar festgehalten wird, um distale Bewegung des Zwischengriffs (150) zu verhindern.

3. Abgabekatheter-Griff-Anordnung (100) nach Anspruch 2, wobei der Griffverriegelungsmechanismus (152, 152') mit dem proximalen Griff (130) und dem Zwischengriff (150) einheitlich geformt ist.

4. Abgabekatheter-Griff-Anordnung (100) nach Anspruch 1, ferner mit:
einem Verschiebe- und Arretiermechanismus (136, 154, 156, 152, 152'), wobei der Zwischengriff (150) in den proximalen Griff zurückgezogen wird, um eine einheitliche Rückzugsposition zu bilden, die so konfiguriert ist, dass sie Bewegung zwischen dem proximalen Griff (130) und dem Zwischengriff (150) begrenzt.

5. Abgabekatheter-Griff-Anordnung (100) nach Anspruch 4, wobei der Zwischengriff (150) einen Lösemechanismus (158) so aufweist, dass der Zwischengriff (150) gelöst und relativ entfernt vom proximalen Griff (130) positioniert werden kann, um Nachladen einer Prothese (300) zu ermöglichen.

6. Abgabekatheter-Griff-Anordnung (100) nach Anspruch 4, wobei der Verschiebe- und Arretiermechanismus (136, 154, 156, 152, 152') mit dem proximalen Griff (130) und dem Zwischengriff (150) einheitlich geformt ist.

7. Abgabekatheter-Griff-Anordnung (100) nach Anspruch 1, wobei der Zwischengriff (150) einen Mechanismus (154, 154a) an einer distalen Position (155') aufweist, der so konfiguriert ist, dass er unbeabsichtigten Rückzug verhindert.

8. Abgabekatheter-Griff-Anordnung (100) nach Anspruch 1, wobei der Zwischengriff (150) mindestens einen vorstehenden Finger zum verriegelnden Verbinden mit dem proximalen Griff (130) aufweist.

9. Abgabekatheter-Griff-Anordnung (100) nach Anspruch 1, wobei der Zwischengriff (150) eine Greiffläche aufweist, vorzugsweise Flügel (169) oder einen Softgrip.

10. Abgabekatheter-Griff-Anordnung (100) nach Anspruch 1, ferner mit einem Führungsdrahtport.

11. Abgabekatheter-Griff-Anordnung (100) nach Anspruch 1, wobei der proximale Griff (130) Vorsprünge (127) aufweist, um für Reibungskraft zu sorgen, die den Zwischengriff (150) während des Prothesenladeablaufs festhält.

12. Abgabekatheter-Griff-Anordnung (100) nach Anspruch 1, wobei der proximale Griff (130) einen Mechanismus zum verriegelnden Erfassen des Zwischengriffs (150) während des Prothesenladeablaufs aufweist.

13. Abgabekatheter-Griff-Anordnung (100) nach Anspruch 1, wobei in einem Verpackungszustand die Relativbewegung der Griffe (130, 150, 170) mechanisch begrenzt ist.

14. Abgabekatheter-Griff-Anordnung (100) nach Anspruch 1, wobei der Zwischengriff (150) ferner einen Arretiermechanismus zum Verbinden mit dem proximalen Griff (130) aufweist, wobei der Arretiermechanismus einen Lösemechanismus hat, der versenkt ist.

15. Verfahren zum Laden einer Prothese (300) auf ein Abgabesystem (100) wobei das Verfahren aufweist:
Bereitstellen eines Prothesenabgabesystems (100) mit einem proximalen Griff (130), der an einem Innenteil (120) angebracht ist, einem Zwischengriff (150), der an einem Zwischenteil (140) angebracht ist, wobei eine Komponente aus dem proximalen und dem Zwischengriff (130, 150) so gestaltet ist, dass sie die andere Komponente aus dem proximalen und dem Zwischengriff (130, 150) unterbringt und ergreift, und einem distalen Griff (170), er an einem Außenteil (160) angebracht ist, das über mindestens einem Abschnitt des Zwischenteils (140) liegt, wobei der proximale Griff (130) einen lösbaren Arretiermechanismus zum lösbaren Ergreifen des Zwischengriffs (150) hat; Bereitstellen einer Prothese (300) in einem nicht eingespannten Zustand;
Positionieren des Zwischengriffs (150) in einer vom proximalen Griff (130) entfernten Position und Laden der Prothese (300) in einen Prothesenkorb (200), wobei der Korb (200) ein entgegengesetztes proximales und distales Ende hat und das proximale Ende des Korbs am distalen Ende des Zwischengriffs (150) fest angeordnet werden kann;
distales Bewegen des distalen Griffs (170), um die Prothese (300) im Außenteil (160) einzuspannen; und
Zurückziehen des Prothesenkorbs (200) in das axial längliche Zwischenteil (140) durch Verschieben des Zwischengriffs (150) in proximaler Richtung, bis er über den lösbaren Arretiermechanismus im proximalen Griff (130) arretiert ist und der Korb (200) nicht mehr mit der Prothese (300) in Kontakt steht,
**dadurch gekennzeichnet, dass**
der proximale Griff (130) eine Öffnung (132) an seinem distalen Ende (129) aufweist, die eine Aufnahme (134) bildet, die sich in den proximalen Griff (130) erheblich erstreckt, und
der Zwischengriff (150) einen proximalen Schaftabschnitt (155, 155') und einen distalen Flanschabschnitt (157a) aufweist,
wobei der proximale Schaftabschnitt (155, 155') des Zwischengriffs (150) in die Aufnahme (134) des proximalen Griffs (130) und aus ihr verschiebbar ist und
wobei der distale Flanschabschnitt (157a) als Anschlag zur Einführung des Zwischengriffs (150) in die Aufnahme (134) des proximalen Griffs (130) dient.

## Revendications

1. Ensemble de poignées de cathéter de pose (100) comprenant :
une poignée proximale (130) fixée à une extrémité proximale (117) d'un élément interne axialement allongé (120) ;
une poignée intermédiaire (150) fixée à une extrémité proximale (137) d'un élément intermédiaire axialement allongé (140) ; et
une poignée distale (170) fixée à une extrémité proximale (157) d'un élément externe axialement allongé (160) recouvrant au moins une partie dudit élément intermédiaire (140),
l'ensemble comprend en outre un panier (200) avec des extrémités proximale et distale opposées, dans lequel ladite extrémité proximale du panier peut être disposée de manière fixe sur l'extrémité distale de la poignée intermédiaire (150),
**caractérisé en ce qu'**au moins l'une desdites poignées proximale et intermédiaire (130, 150) est conçue pour loger et mettre en prise l'autre desdites poignées proximale et intermédiaire (130, 150),
ladite poignée proximale (130) comprend une ouverture (132) au niveau de son extrémité distale (129) définissant un réceptacle (134) s'étendant de manière significative dans ladite poignée proximale (130), et
ladite poignée intermédiaire (150) comprend une partie de tige proximale (155, 155') et une partie de rebord distale (157a),
dans lequel ladite partie de tige proximale (155, 155') de ladite poignée intermédiaire (150) peut coulisser à l'intérieur et à l'extérieur dudit réceptacle (134) de ladite poignée proximale (130), et
dans lequel ladite partie de rebord distale (157a) sert de butée pour l'insertion de ladite poignée intermédiaire (150) dans ledit réceptacle (134) de ladite poignée proximale (130).

2. Ensemble de poignées de cathéter de pose (100) selon la revendication 1, dans lequel ladite poignée proximale (130) et ladite poignée intermédiaire (150) comprennent un mécanisme de verrouillage de poignée (152, 152'), une partie de ladite poignée intermédiaire (150) étant retenue de manière amovible par ladite poignée proximale (130) pour empêcher le mouvement distal de ladite poignée intermédiaire (150).

3. Ensemble de poignées de cathéter de pose (100) selon la revendication 2, dans lequel ledit mécanisme de verrouillage de poignée (152, 152') est moulé de manière unitaire avec ladite poignée proximale (130) et ladite poignée intermédiaire (150).

4. Ensemble de poignées de cathéter de pose (100) selon la revendication 1, comprenant en outre :
un mécanisme de coulissement et de blocage (136, 154, 156, 152, 152'), dans lequel ladite poignée intermédiaire (150) est rétractée dans la poignée proximale afin de définir une position rétractée unitaire configurée pour limiter le déplacement entre ladite poignée proximale (130) et ladite poignée intermédiaire (150).

5. Ensemble de poignées de cathéter de pose (100) selon la revendication 4, dans lequel ladite poignée intermédiaire (150) comprend un mécanisme de libération (158) de sorte que la poignée intermédiaire (150) peut être libérée et positionnée relativement à distance de ladite poignée proximale (130) pour permettre de recharger une prothèse (300).

6. Ensemble de poignées de cathéter de pose (100) selon la revendication 4, dans lequel ledit mécanisme de coulissement et de blocage (136, 154, 156, 152, 152') est moulé de manière unitaire avec ladite poignée proximale (130) et ladite poignée intermédiaire (150).

7. Ensemble de poignées de cathéter de pose (100) selon la revendication 1, dans lequel ladite poignée intermédiaire (150) comprend un mécanisme (154, 154a) dans une position distale (155') configurée pour empêcher la rétraction involontaire.

8. Ensemble de poignées de cathéter de pose (100) selon la revendication 1, dans lequel ladite poignée intermédiaire (150) comprend au moins un doigt en saillie pour le raccordement par verrouillage avec ladite poignée proximale (130).

9. Ensemble de poignées de cathéter de pose (100) selon la revendication 1, dans lequel ladite poignée intermédiaire (150) comprend une surface de préhension, de préférence des ailes (169) ou une prise souple.

10. Ensemble de poignées de cathéter de pose (100) selon la revendication 1, comprenant en outre un orifice de fil-guide.

11. Ensemble de poignées de cathéter de pose (100) selon la revendication 1, dans lequel ladite poignée proximale (130) comprend des saillies (127) pour fournir la force de friction retenant ladite poignée intermédiaire (150) pendant le procédé de chargement de la prothèse.

12. Ensemble de poignées de cathéter de pose (100) selon la revendication 1, dans lequel ladite poignée proximale (130) comprend un mécanisme pour capturer par verrouillage ladite poignée intermédiaire (150) pendant le procédé de chargement de la prothèse.

13. Ensemble de poignées de cathéter de pose (100) selon la revendication 1, dans lequel, dans une condition ainsi emballée, le mouvement relatif des poignées (130, 150, 170) est mécaniquement limité.

14. Ensemble de poignées de cathéter de pose (100) selon la revendication 1, dans lequel ladite poignée intermédiaire (150) comprend en outre un mécanisme de blocage pour raccorder ladite poignée proximale (130), ledit mécanisme de blocage ayant un mécanisme de libération qui est enfoncé.

15. Procédé pour charger une prothèse (300) sur un système de pose (100), le procédé comprenant les étapes consistant à :
prévoir un système de pose de prothèse (100) comprenant une poignée proximale (130) fixée à un élément interne (120), une poignée intermédiaire (150) fixée sur un élément intermédiaire (140), l'une desdites poignées proximale et intermédiaire (130, 150) étant conçue pour loger et mettre en prise une autre parmi lesdites poignées proximale et intermédiaire (130, 150), et une poignée distale (170) fixée à un élément externe (160) recouvrant au moins une partie dudit élément intermédiaire (140), ladite poignée proximale (130) ayant un mécanisme de blocage amovible pour mettre en prise de manière amovible ladite poignée intermédiaire (150) ;
prévoir une prothèse (300) dans une condition non contrainte ;
positionner ladite poignée intermédiaire (150) dans une position à distance de ladite poignée proximale (130) et charger ladite prothèse (300) dans un panier de prothèse (200), dans lequel ledit panier (200) ayant des extrémités proximale et distale opposées et ladite extrémité proximale du panier peut être disposé de manière fixe sur l'extrémité distale de la poignée intermédiaire (150) ;
déplacer ladite poignée distale (170) de manière distale pour contraindre ladite prothèse (300) à l'intérieur dudit élément externe (160) ; et
rétracter ledit panier de prothèse (200) dans ledit élément intermédiaire axialement allongé (140) en faisant coulisser ladite poignée intermédiaire (150) dans une direction proximale jusqu'à ce qu'il se bloque dans ladite poignée proximale (130) via ledit mécanisme de blocage amovible et que ledit panier (200) ne soit plus en contact avec ladite prothèse (300),
**caractérisé en ce que** :
ladite poignée proximale (130) comprend une ouverture (132) au niveau de son extrémité distale (129) définissant un réceptacle (134) s'étendant de manière significative dans ladite poignée proximale (130), et
ladite poignée intermédiaire (150) comprend une partie de tige proximale (155, 155') et une partie de rebord distale (157a),
dans lequel ladite partie de tige proximale (155, 155') de ladite poignée intermédiaire (150) peut coulisser à l'intérieur et à l'extérieur dudit réceptacle (134) de ladite poignée proximale (130), et
dans lequel ladite partie de rebord distale (157a) sert de butée pour l'insertion de ladite poignée intermédiaire (150) dans ledit réceptacle (134) de ladite poignée proximale (130).
